(19) Europäisches Patentamt
European Patent Office
Office européen des brevets

(11) Publication number: 0 158 350
B1

(12) EUROPEAN PATENT SPECIFICATION

(45) Date of publication of patent specification: 08.03.89

(51) Int. Cl.⁴: C 01 B 33/26

(21) Application number: 85104392.7

(22) Date of filing: 11.04.85

# (54) Quinary and senary molecular sieve compositions.

(30) Priority: 13.04.84 US 600168
13.04.84 US 600181
13.04.84 US 600182
13.04.84 US 600183

(43) Date of publication of application:
16.10.85 Bulletin 85/42

(45) Publication of the grant of the patent:
08.03.89 Bulletin 89/10

(84) Designated Contracting States:
BE DE FR GB IT NL

(58) References cited:
EP-A-0 103 117

(73) Proprietor: UNION CARBIDE CORPORATION
39 Old Ridgebury Road
Danbury Connecticut 06817 (US)

(72) Inventor: Lok, Brent Mei Tak
17A Deerwood Drive
New City New York 10956 (US)
Inventor: Marcus, Bonita Kristoffersen
49 Meadow Place
Rye New York 10580 (US)
Inventor: Flanigen, Edith Marie
502 Woodland Hills Road
White Plains New York 10603 (US)

(74) Representative: Eggert, Hans-Gunther, Dr.
Räderscheidtstrasse 1
D-5000 Köln 41 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 158 350 B1

**Description**

The invention relates to a novel class of crystalline three-dimensional microporous molecular sieves, to the method of their preparation and to their use as adsorbents and catalysts. The invention relates to novel quinary and senary molecular sieves having framework tetrahedral oxide units of two or three elements 'M" ($MO_2^n$), in addition to aluminum ($AlO_2^-$), phosphorus ($PO_2^+$) and silicon ($SiO_2$) where "M" denotes the elements as hereinafter discussed and "n" is −3, −2, −1, 0 or +1.

Molecular sieves of the crystalline aluminosilicate zeolite type are well known in the art and now comprise over 150 species of both naturally occuring and synthetic compositions. In general the crystalline zeolites are formed from corner-sharing $A1O_2$ and $SiO_2$ tetrahedra and are characterized by having pore openings of uniform dimensions, having a significant ion-exchange capacity and being capable of reversibly desorbing an adsorbed phase which is dispersed throughout the internal voids of the crystal without displacing any atoms which make up the permanent crystal structure.

Other crystalline microporous compositions which are not zeolite, i.e. do not contain $A1O_2$ tetrahedra as essential framework constituents, but which exhibit the ion-exchange and/or adsorption characteristics of the zeolites are also known. Metal organosilicates which are said to possess ion-exchange properties, have uniform pores and are capable of reversibly adsorbing molecules having molecular diameters of about 6Å or less, are reported in U.S. Patent No. 3,941,871 issued March 2, 1976 to Dwyer et al. A pure silica polymorph, silicalite, having molecular sieving properties and a neutral framework containing neither cations nor cation sites is disclosed in U.S. Patent No. 4,061,724 issued December 6, 1977 to R. W. Grose et al.

A recently reported class of microporous compositions and the first framework oxide molecular sieves synthesized without silica, are the crystalline aluminophosphate compositions disclosed in U.S. Patent No. 4,310,440 issued January 12, 1982, to Wilson et al. These materials are formed from $A1O_2$ and $PO_2$ tetrahedra and have electrovalently neutral frameworks as in the case of silica polymorphs. Unlike the silica molecular sieve, silicalite, which is hydrophobic due to the absence of extra-structural cations, the aluminophosphate molecular sieves are moderately hydrophilic, apparently due to the difference in electronegativity between aluminum and phosphorus. Their intracrystalline pore volumes and pore diameters are comparable to those known for zeolites and silica molecular sieves.

In U.S. Patent No. 4,400,871 which corresponds to EP—A—103 117 are disclosed silicoaluminophosphates which are both microporous and crystalline. The materials have a three dimensional crystal framework of $PO_2^+$, $AlO_2^-$ and $SiO_2$ tetrahedral units and, exclusive of any alkali metal or calcium which may optically be present, as as-synthesized empirical chemical composition on an anhydrous basis of:

$$mR:(Si_xAL_yP_z)O_2$$

wherein "R" represents at least one organic templating agent present in the intracrystalline pore system; "M" represents the moles of "R" present per mole of $(Si_xAL_yP_z)O_2$ and has a value of from zero to 0.3; the maximum value in each case depending upon the molecular dimensions of the templating agent and the available void volume of the pore system of the particular silicoaluminophosphate species involved; and "x", "y", and "z" represent the mole fractions of silicon, aluminum and phosphorus, respectively, present as tetrahedral oxides. The minimum value of each of "x", "y", and "z" is 0.01 and preferably 0.02. The maximum value for "x" is 0.98; for "y" is 0.60; and for "z" is 0.52. These silicoaluminophosphates exhibit several physical and chemical properties which are characteristic of aluminosilicate zeolites and aluminophosphates.

In EP—A—121232 there is described a novel class of titanium-containing molecular sieves whose chemical composition in the as-synthesized and anhydrous form is represented by the unit empirical formula:

$$mR:(Ti_xAL_yP_z)O_2$$

wherein "R" represents at least one organic templating agent present in the intracrystalline pore system; "m" represents the moles of "R" present per mole of $(Ti_xAL_yP_z)O_2$ and has a value of between zero and about 5.0; and "x", "y", and "z" represent the mole fractions of titanium, aluminum and phosphorus, respectively, present as tetrahedral oxides.

In EP—A—132708 there is described a novel class of crystalline metal aluminophosphates having three-dimensional microporous framework structures of $MO_2$, $A1O_2$ and $PO_2$ tetrahedral units and having an empirical chemical composition on an anhydrous basis expressed by the formula:

$$mR:(M_xAL_yP_z)O_2$$

wherein "R" represents at least one organic templating agent present in the intracrystalline pore system; "m" represents the moles of "R" present per mole of $(M_xAL_yP_z)O_2$ and has a value of from zero to 0.3; "M" represents at least one metal of the group magnesium, manganese, zinc and cobalt; and "x", "y", and "z"

represent the mole fraction of the metal "M", aluminum and phosphorus, respectively, present as tetrahedral oxides.

In EP—A—131946 application Serial No. 514,335, filed July 15, 1983, there is described a novel class of crystalline ferroaluminophosphates having a three-dimensional microporous framework structure of $FeO_2$, $AlO_2$ and $PO_2$ tetrahedral units and having an empirical chemical composition on an anhydrous basis expressed by the formula

$$mR:(Fe_xAL_yP_z)O_2$$

wherein "R" represents at least one organic templating agent present in the intracrystalline pore system; "m" represents the moles of "R" present per mole of $(Fe_xAL_yP_z)O_2$ and has a value of from zero to 0.3; and "x", "y", and "z" represent the mole fraction of the iron, aluminum and phosphorus, respectively, present as tetrahedral oxides.

The instant invention relates to molecular sieve compositions having framework tetrahedral oxide units of $MO_2^n$, $AlO_2^-$, $PO_2^+$ and $SiO_2$ where "$MO_2^n$" represents tetrahedral oxide units of two or three different elements, and "n" is −3, −2, −1, 0 or +1.

Fig. 1 is a ternary diagram wherein parameters relating to the instant compositions are set forth as mole fractions.

Fig. 2 is a ternary diagram wherein parameters relating to the instant compositions are set forth as mole fractions.

Fig. 3 is a ternary diagram wherein parameters relating to the reaction mixtures employed in the preparation of the compositions of this invention are set forth as mole fractions.

The instant invention relates to a new class of quinary and senary crystalline molecular sieves having three dimensional microporous framework structures of $MO_2^n$, $AlO_2^-$, $PO_2^+$ and $SiO_2$ tetrahedral oxide units, where "n" is −3, −2, −1, 0 or +1. These new molecular sieves exhibit ion-exchange, adsorption and catalytic properties and, accordingly, find wide use as adsorbents and catalysts. The members of this novel class of compositions have an empirical chemical composition on an anhydrous basis expressed by the formula:

$$mR: (M_wAl_xP_ySi_z)O_2$$

wherein "R" represents at least one organic templating agent present in the intracrystalline pore system; "m" represents the molar amount of "R" present per mole of $(M_wAl_xP_ySi_z)O_2$, and has a value of from zero (0) to about 0.3; "M" represents at least two elements selected from group consisting of arsenic, beryllium, boron, chromium, cobalt, gallium, germanium, iron, lithium, magnesium, manganese, titanium, vanadium, and zinc; "n" may be −3, −2, −1, 0 or +1; and "w", "x", "y", and "z" represent the mole fractions of elements "M", aluminum, phosphorus, silicon, respectively, present as tetrahedral oxides, each having a value of at least 0.01.

The molecular sieves of the instant invention will be generally referred to herein by the acronym "SENAPSO" to designate framework structures of at least two elements having tetrahedral oxide units "$MO_2^n$" and having $AlO_2^-$, $PO_2^+$ and $SiO_2$ tetrahedral oxide units, where "n" is −3, −2, −1, 0 or +1. Actual class members will be identified by denominating the various structural species which make up each of the class by assigning a number are are identified as "SENAPSO-i" wherein "i" is an integer. Actual class members will be identified by replacing the "SEN" of "SENAPSO-i" with the elements "M" present in the given SENAPSO such that the framework elements present in the tetrahedral oxide form are identified. These designations are arbitrary ones and are not intended to denote structural relationship to another material(s) which may also be characterized by a numbering system.

The instant invention relates to a new class of crystalline molecular sieves having a three-dimensional microporous crystal framework structure of $MO_2^n$, $AlO_2^-$, $PO_2^+$ and $SiO_2$ tetrahedral oxide units, where "n" is −3, −2, −1, 0 or +1. These new molecular sieves exhibit ion-exchange adsorption and catalytic properties and accordingly find wide use as adsorbents and catalysts.

The crystalline molecular sieves of the instant invention have three-dimensioned mircoporous framework structures of $MO_2^n$, $AlO_2^-$, $PO_2^+$ and $SiO_2$ tetrahedral oxide units and have an empirical chemical composition on an anhydrous basis expressed by the formula:

$$mR: (M_wAl_xP_ySi_z)O_2$$

wherein "R" represents at least one organic templating agent present in the intracrystalline pore system; "m" represents the molar amount of "R" present per mole of $(M_wAl_xP_ySi_z)O_2$, and has a value of from zero to about 0.3; "M" represents at least two elements selected from the group consisting of arsenic, beryllium, boron, chromium, cobalt, gallium, germanium, iron, lithium, magnesium, manganese, titanium, vanadium, and zinc; "n" may be −3, −2, −1, 0 or +1 depending on the oxidation state of "M"; and "w", "x", "y", and "z" represent the mole fractions of elements "M", aluminum, phosphorus and silicon, respectively, present as tetrahedral oxides. The mole fractions "w", "x", "y" and "z" are generally defined as being within the pentagonal compositional area defined by points, A, B, C, D and E of Fig. 1 where "M" denotes at least two

different elements, as above identified and "w" denotes the combined mole fractions of the elements such that "w" = "$w_1$" + "$w_2$" + "$w_3$" and etc. and each element has a mole fraction of at least 0.01. In one embodiment two elements "M" are present and in another embodiment three elements "M" are present. Points A, B, C, D and E of Fig. 1 have the following values for "w", "x", "y", and "z":

| | Mole Fraction | | |
|---|---|---|---|
| Point | x | y | (z+w) |
| A | 0.60 | 0.37 | 0.03 |
| B | 0.37 | 0.60 | 0.03 |
| C | 0.01 | 0.60 | 0.39 |
| D | 0.01 | 0.01 | 0.98 |
| E | 0.60 | 0.01 | 0.39 |

In a preferred subclass of the SENAPSO molecular sieves the values of "w", "x", "y", and "z" in the above formula are within the hexagonal compositional area defined by points a, b, c, d, e and f of Fig. 2. Points a, b, c, d, e and f have the following values for "w", "x", "y", and "z".

| | Mole Fraction | | |
|---|---|---|---|
| Point | x | y | (w+z) |
| a | 0.60 | 0.37 | 0.03 |
| b | 0.37 | 0.60 | 0.03 |
| c | 0.01 | 0.60 | 0.39 |
| d | 0.01 | 0.39 | 0.60 |
| e | 0.39 | 0.01 | 0.60 |
| f | 0.60 | 0.01 | 0.39 |

The SENAPSOs of the invention are useful as adsorbents, catalysts, ion-exchangers, and the like in much the same fashion as aluminosilicates have been employed heretofore, although their chemical and physical properties are not necessarily similar to those observed for aluminosilicates.

SENAPSO compositions are generally synthesized by hydrothermal crystallization from a reaction mixture containing reactive sources of elements "M", aluminum, phosphorus and silicon, and preferably an organic templating agent, i.e. structure-directing agent. The structure-directing agents are preferably a compound of an element of Group VA of the Periodic Table, and/or optionally an alkali or other metal. The reaction mixture is generally placed in a sealed pressure vessel, preferably lined with an inert plastic material such as polytetrafluoroethylene and heated, preferably under autogeneous pressure at an effective temperature which is preferably between 50°C and 250°C, and more preferably between 100°C and 200°C until crystals of the SENAPSO product are obtained, usually over an effective crystallization time of from several hours to several weeks. Typical crystallization times are from about 2 hours to about 30 days with from about 4 hours to about 20 days generally being employed to obtain SENAPSO products. The product is recovered by any convenient method such as centrifugation or filtration.

In synthesizing the SENAPSO compositions of the instant invention, it is preferred to employ reaction mixture composition expressed in terms of the molar ratios as follows:

$$aR: (M_wAl_xP_ySi_z)O_2:bH_2O$$

wherein "R" is an organic templating agent: "a" is the amount of organic templating agent "R" and has a value from zero to about 6 and is preferably an effective amount within the range of greater than zero (0) to about 6, and more preferably from greater than zero to about 2; "b" has a value of from zero (0) to about 500, preferably between about 2 and about 300; "M" is as above defined; and "w", "x", "y", and "z" represent the mole fractions of elements "M", aluminum, phosphorus and silicon, respectively, and each has a value of at least 0.01 with the proviso that each "M" has a value of at least 0.01.

In a preferred embodiment the reaction mixture is selected such that the mole fractions "w", "x", "y", and "z" are generally defined as being within the pentagonal compositional area defined by points F, G, H, I and J of the ternary diagram of Fig. 3. Points F, G, H, I and J of Fig. 3 have the following values for "w", "x", "y", and "z".

| | Mole Fraction | | |
|---|---|---|---|
| Point | x | y | (z+w) |
| F | 0.60 | 0.37 | 0.03 |
| G | 0.37 | 0.60 | 0.03 |
| H | 0.01 | 0.60 | 0.39 |
| I | 0.01 | 0.01 | 0.98 |
| J | 0.60 | 0.01 | 0.39 |

For reasons unknown at present, not every reaction mixture gives crystalline SENAPSO products when reaction products were examined for SENAPSO products by X-ray analysis. Reaction mixtures from which crystalline SENAPSO products are obtained are reported in the examples hereinafter as numbered examples and those reaction mixtures from which SENAPSO products are not identified by use of X-ray analysis are reported as lettered examples.

In the foregoing expression of the reaction composition, the reactants are normalized with respect to the total of "w", "x", "y", and "z" such that $(w+x+y+z) = 1.00$ mole, whereas in the examples the reaction mixtures may be expressed in terms of molar oxide ratios and may be normalized to the moles of $P_2O_5$. This latter form is readily converted to the former form by routine calculations by dividing the number of moles of each component (including the template and water) by the total number of moles of each "M", aluminum, phosphorus and silicon which results in normalized mole fractions based on total moles of the aforementioned components. The template and water are normalized similarly by dividing by the total number of moles of each "M", aluminum, phosphorus and silicon.

In forming reaction mixtures from which the instant molecular sieves are formed the organic templating agent, i.e., "template", can be any of those heretofore proposed for use in the synthesis of conventional zeolite aluminosilicates. In general these compounds contain elements of Group VA of the Periodic Table of Elements, particularly nitrogen, phosphorus, arsenic and antimony, preferably nitrogen or phosphorus and most preferably nitrogen, which compounds also contain at least one alkyl or aryl group having from 1 to 8 carbon atoms. Particularly preferred compounds for use as templating agents are the amines, quaternary phosphonium compounds and quaternary ammonium compounds, the latter two being represented generally by the formula $R_4X^+$ wherein "X" is nitrogen or phosphorus and each R is an alkyl or aryl group containing from 1 to 8 carbon atoms. Polymeric quaternary ammonium salts such as $[(C_{14}H_{32}N_2)(OH)_2]_x$ wherein "x" has a value of at least 2 are also suitably employed. The mono-, di- and triamines are advantageously utilized, either alone or in combination with a quaternary ammonium compound or other templating compound. Mixtures of two or more templating agents can either produce mixtures of the desired SENAPSOs or the more strongly directing templating species may control the course of the reaction with the other templating species serving primarily to establish the pH conditions of the reaction gel. Representative templating agents include: tetramethylammonium; tetraethylammonium; tetrapropylammonium, tetrabutylammonium ions; tetrapentylammonium ions; di-n-propylamine; tri-n-propylamine, triethylamine; triethanolamine; piperidine; cyclohexylamine; 2-methylpyridine; N,N-dimethylbenzylamine; N,N-dimethylethanolamine; chlorine; N,N-dimethylpiperazine; 1,4-diazabicyclo (2,2,2,) octane; N-methyldiethanolamine, N-methylethanolamine; N-methylpiperidine; 3-methylpiperidone; N-methylcyclohexylamine; 3-methylpyridine; 4-methylpyridine; quinuclidine; N,N'-dimethyl-1,4-diazabicyclo (2,2,2,) octane ion; di-n-butylamine; neopentylamine; di-n-pentylamine; isopropylamine; t-butylamine; ethylenediamine; pyrrolidine; and 2-imidazolidone. Not every templating agent will direct the formation of every species of SENAPSO, i.e., a single templating agent may, with proper manipulation of the reaction conditions, direct the formation of several SENAPSO compositions, and a given SENAPSO composition may be produced using several different templating agents.

Most any reactive silicon source may be employed such that $SiO_2$ tetrahedral oxide units are formed *in situ.* The reactive source of silicon may be silica, either as a silica sol or as fumed silica, a reactive solid amorphous precipitated silicas, silica gel, alkoxides of silicon, silicic acid or alkali metal silicate and mixtures thereof.

The most suitable phosphorus source yet found for the present process is phosphoric acid, but organic phosphates such as triethyl phosphate have been found satisfactory, and so also have crystalline or amorphous aluminophosphates such as the $AlPO_4$ composition of US—A—4,310,440. Organo-phosphorus compounds, such as tetrabutylphosphonium bromide, do not apparently serve as reactive sources of phosphorus, but these compounds do function as templating agents. Conventional phosphorus salts such as sodium metaphosphate, may be used, at least in part, as the phosphorus source, but are not preferred.

The preferred aluminum source is either an aluminum alkoxide, such as aluminum isoproproxide, or pseudoboehmite. The crystalline or amorphous aluminophosphates which are a suitable source of phosphorus are, of course, also suitable sources of aluminum. Other sources of aluminum used in zeolite synthesis, such as gibbsite, sodium aluminate and aluminum trichloride, can be employed but are not preferred.

Reactive sources of elements "M" (arsenic, beryllium, boron, chromium, cobalt, gallium, germanium, iron, lithium, magnesium, manganese, titanium, vanadium, and/or zinc) can be introduced into the reaction system in any form which permits the formation *in situ* of the framework tetrahedral units. Compounds which may be employed include oxides, hydroxides and alkoxides and salts such as halides, nitrates, sulfates, carboxylates (e.g. acetates) and mixtures of the above compounds.

While not essential to the synthesis of SENAPSO compositions, stirring or other moderate agitation of the reaction mixture and/or seeding the reaction mixture with seed crystals of either the SENAPSO species to be produced or a topologically similar aluminophosphate, aluminosilicate or molecular sieve composition, facilitates the crystallization procedure.

After crystallization the SENAPSO product may be isolated and advantageously washed with water and dried in air. The as-synthesized SENAPSO generally contains within its internal pore system at least one form of the templating agent, referred to herein as the "organic moiety", employed in its formation.

Most commonly the organic moiety is present, at least in part, as a charge-balancing cation as is generally the case with as-synthesized aluminosilicate zeolites prepared from organic-containing reaction systems. It is possible, however, that some or all of the organic moiety is an occluded molecular species in a particular SENAPSO species. As a general rule the templating agent, and hence the occluded organic species, is too large to move freely through the pore system of the SENAPSO product and must be removed by calcining the SENAPSO at temperatures of 200°C to 700°C to thermally degrade the organic species. In a few instances the pores of the SENAPSO product are sufficiently large to permit transport of the templating agent, particularly if the latter is a small molecule, and accordingly complete or partial removal thereof can be accomplished by conventional desorption procedures such as carried out in the case of zeolites. It will be understood that the term "as-synthesized" as used herein does not include the condition of the SENAPSO species wherein the organic moiety occupying the intracrystalline pore system as a result of the hydrothermal crystallization process has been reduced by post-synthesis treatment such that the value of "m" in the composition formula:

$$mR: (M_wAl_xP_ySi_z)O_2$$

has a value of less than 0.02. The other symbols of the formula are as defined hereinabove. in those preparations in which an alkoxide is employed as the source of elements "M", aluminum, phosphorus or silicon, the corresponding alcohol is necessarily present in the reaction mixture since it is a hydrolysis product of the alkoxide. It has not been determined whether this alcohol participates in the synthesis process as a templating agent. For the purposes of this application, however, this alcohol is arbitrarily omitted from the class of templating agents, even if it is present in the as-synthesized SENAPSO material.

Since the present SENAPSO compositions are formed from $MO_2^n$, $AlO_2^-$, $PO_2$ and $SiO_2$ tetrahedral units which, respectively, have a net charge of "n" −1, +1 and 0, the matter of cation exchangeability is considerably more complicated than in the case of zeolitic molecular sieves in which, ideally, there is a stoichiometric relationship between $AlO_2^-$ tetrahedra and charge-balancing cations. In the instant compositions, an $AlO_2^-$ tetrahedron can be balanced electrically either by association with a $PO_2^+$ tetrahedron or a simple cation such as an alkali metal cation, a proton ($H^+$), cations of elements "M" present in the reaction mixture, or an organic cation derived from the templating agent. Similarly, $MO_2^n$ tetrahedra (where "n" may be −3, −2, −1, 0 or +1) can be balanced electrically by association with $PO_2^+$ tetrahedra, a simple cation such as an alkali metal cation, a proton ($H^+$), cations of elements "M" present in the reaction mixture, organic cations derived from the templating agent, or other divalent or polyvalent metal cations introduced from an extraneous source. It has also been postulated that non-adjacent $AlO_2^-$ and $PO_2^+$ tetrahedral pairs can be balanced by $Na^+$ and $OH^-$ respectively [Flanigen and Grose, Molecular Sieve Zeolites-I, ACS, Washington, DC (1971)].

The SENAPSO compositions of the present invention may exhibit catio-exchange capacity when analyzed using ion-exchange techniques heretofore employed with zeolitic aluminosilicates and have pore diameters which are inherent in the lattice structure of each species and which are at least about 3Å in diameter. Ion exchanges of SENAPSO compositions is ordinarily possible only after organic moiety derived from the template, present as a result of synthesis, has been removed from the pore system. Dehydration to remove water present in the as-synthesized SENAPSO compositions can usually be accomplished, to some degree at least, in the usual manner without removal of the organic moiety, but the absence of the organic species greatly facilitates adsorption and desorption procedures. As illustrated hereinafter, the SENAPSO materials have various degrees of hydrothermal and thermal stability, some being quite remarkable in this regard, and function well as molecular sieve adsorbents and hydrocarbon conversion catalysts or catalyst bases.

In each example the stainless steel reaction vessel utilized was lined with the inert plastic material, polytetrafluoroethylene, to avoid contamination of the reaction mixture. In general, the final reaction mixture from which each SENAPSO composition is crystallized is prepared by forming mixtures of less than all of the reagents and thereafter incorporating into these mixtures addition reagents either singly or in the form of other intermediate mixtures of two or more reagents. In some instance the reagents admixed retain their identity in the intermediate mixture and in other cases some or all of the reagants are involved in chemical reactions to produce new reagents. The term "mixture" is applied in both cases. Further, unless otherwise specified, each intermediate mixture as well as the final reaction mixture was stirred until substantially homogeneous.

X-ray analysis of reaction products are obtained by X-ray analysis using standard X-ray powder diffraction techniques. The radiation source is a high-intensity, copper target, X-ray tube operated at 50 KV and 40mA. The diffraction pattern from the copper K-alpha radiation and graphite monochromator is suitably recorded by an X-ray spectrometer scintillation counter, pulse height analyzer and strip chart recorder. Flat compressed powder samples are scanned at 2° (2 theta) per minute, using a two second time constant. Interplanar spacings (d) in Angstrom units are obtained from the position of the diffraction peaks expressed as 2θ where θ is the Bragg angle as observed on the strip chart. Intensities are determined from the heights of diffraction peaks after subtracting background, "$I_o$" being the intensity of the strongest line or peak, and "I" being the intensity of each of the other peaks. Alternatively, the X-ray patterns are obtained from the copper K-alpha radiation by use of computer based techniques using Siemens D—500 X-ray

powder diffractometers and Siemens Type K—805 X-ray sources, available from Siemens Corporation, Cherry Hill, New Jersey, with appropriate computer interface.

As will be understood by those skilled in the art the determination of the parameter 2 theta is subject to both human and mechanical error, which in combination, can impose an uncertainty of about ±0.4° (denotes plus or minus 0.4°) on each reported value of 2 theta. This uncertainty is, of course, also manifested in the reported values of the d-spacings, which are calculated from the 2 theta values. This imprecision is general throughout the art and is not sufficient to preclude the differentiation of the present crystalline materials from each other and from the compositions of the prior art. In some of the X-ray patterns reported, the relative intensities of the d-spacings are indicated by the notations vs, s, m, w and vw which represent very strong, strong, medium, weak and very weak, respectively.

In certain instances the purity of a synthesized product may be assessed with reference to its X-ray powder diffraction pattern. Thus, for example, if a sample is stated to be pure, it is intended only that the X-ray pattern of the sample is free of lines attributable to crystalline impurities not that there are no amorphous materials present.

The molecular sieves of the instant invention may be characterized by their X-ray powder diffraction patterns and such may have one of the X-ray patterns set forth in the following Tables A through N, wherein said X-ray patterns are for both the as-synthesized and calcined forms:

TABLE A (SENAPSO-5)

| 2θ | d (Å) | Relative Intensity |
|---|---|---|
| 7.2 - 7.7 | 12.28 - 11.48 | m-vs |
| 19.4 - 19.9 | 4.58 - 4.46 | w-m |
| 20.85 - 21.3 | 4.26 - 4.17 | w-vs |
| 22.1 - 22.6 | 4.02 - 3.93 | m-vs |
| 25.6 - 26.1 | 3.480 - 3.414 | vw-m |

TABLE B (SENAPSO-11)

| 2θ | d (Å) | Relative Intensity |
|---|---|---|
| 7.8 - 8.2 | 11.19 - 10.85 | m-s |
| 9.0 - 9.8 | 9.83 - 9.03 | vw-vs |
| 12.8 - 13.6 | 6.92 - 6.51 | vw-m |
| 19.9 - 20.5 | 4.46 - 4.33 | m-s |
| 20.8 - 21.8 | 4.27 - 4.08 | m-vs |
| 22.0 - 22.6 | 4.04 - 3.93 | m-vs |
| 22.6 - 23.1 | 3.93 - 3.85 | vw-vs |
| 23.1 - 23.5 | 3.85 - 3.79 | w-vs |

TABLE C (SENAPSO-16)

| 2θ | d (Å) | Relative Intensity |
|---|---|---|
| 11.3 - 11.6 | 7.83 - 7.63 | w-vs |
| 18.55 - 18.9 | 4.78 - 4.70 | vm-m |
| 21.85 - 22.2 | 4.07 - 4.00 | m-vs |
| 22.8 - 23.3 | 3.900 - 3.818 | w-m |
| 26.4 - 27.3 | 3.370 - 3.267 | w-m |
| 29.6 - 29.9 | 3.018 - 2.988 | w-m |

TABLE D (SENAPSO-20)

| 2θ | d (Å) | Relative Intensity |
|---|---|---|
| 13.8 - 14.2 | 6.42 - 6.23 | m-vs |
| 19.6 - 20.15 | 6.53 - 4.41 | m |
| 24.1 - 24.7 | 3.695 - 3.603 | m-vs |
| 27.9 - 28.6 | 3.198 - 3.121 | w |
| 31.3 - 32.05 | 2.861 - 2.791 | w |
| 34.35 - 35.0 | 2.610 - 2.601 | w-m |

TABLE E (SENAPSO-31)

| 2θ | d (Å) | Relative Intensity |
|---|---|---|
| 8.4 - 9.5 | 10.53 - 9.31 | w-s |
| 20.2 - 20.4 | 4.40 - 4.35 | m |
| 22.0 - 22.1 | 4.040 - 4.022 | m |
| 22.5 - 22.7 | 3.952 - 3.92 | vs |
| 31.6 - 31.8 | 2.831 - 2.814 | w-m |

TABLE F (SENAPSO-34)

| 2θ | d (Å) | Relative Intensity |
|---|---|---|
| 9.3 - 9.8 | 9.51 - 9.03 | m-vs |
| 12.6 - 13.2 | 7.03 - 6.71 | w-m |
| 15.8 - 16.3 | 5.61 - 5.44 | vw-m |
| 20.25 - 21.2 | 4.39 - 4.19 | w-vs |
| 24.8 - 25.4 | 3.59 - 3.507 | vw-m |
| 30.0 - 30.9 | 2.979 - 2.894 | vw-m |

TABLE G (SENAPSO-35)

| 2θ | d (Å) | Relative Intensity |
|---|---|---|
| 10.6 - 11.1 | 8.35 - 7.97 | vw-vs |
| 13.1 - 13.7 | 6.76 - 6.46 | vw-vs |
| 17.0 - 17.6 | 5.22 - 5.04 | w-s |
| 20.6 - 21.25 | 4.31 - 4.18 | vw-m |
| 21.6 - 22.3 | 4.11 - 3.99 | m-vs |
| 28.1 - 28.8 | 3.175 - 3.100 | vw-m |

TABLE H (SENAPSO-36)

| 2θ | d (Å) | Relative Intensity |
|---|---|---|
| 7.45 - 8.0 | 11.14 - 11.05 | vs |
| 8.1 - 8.3 | 10.91 - 10.65 | w-m |
| 16.3 - 16.6 | 5.44 - 5.34 | w-m |
| 18.9 - 19.4 | 4.70 - 4.57 | w-m |
| 20.7 - 21.0 | 4.29 - 4.23 | w-m |

TABLE J (SENAPSO-39)

| 2θ | d (Å) | Relative Intensity |
|---|---|---|
| 9.2 - 9.6 | 9.61 - 9.21 | m |
| 13.1 - 13.5 | 6.76 - 6.56 | m |
| 17.8 - 18.4 | 4.98 - 4.82 | w-m |
| 20.8 - 21.3 | 4.27 - 4.17 | m-vs |
| 22.2 - 22.85 | 4.00 - 3.892 | m-vs |
| 26.4 - 27.05 | 3.376 - 3.296 | w-m |

TABLE K (SENAPSO-43)

| 2θ | d (Å) | Relative Intensity |
|---|---|---|
| 12.3 - 12.95 | 7.20 - 6.83 | m-vs |
| 16.8 - 17.45 | 5.28 - 5.09 | vw-w |
| 21.45 - 21.85 | 4.145 - 4.071 | m-vs |
| 27.1 - 27.85 | 3.291 - 3.232 | w-vs |
| 32.4 - 33.2 | 2.763 - 2.699 | vw-m |

TABLE L (SENAPSO-44)

| 2θ | d (Å) | Relative Intensity |
|---|---|---|
| 9.2 - 9.6 | 9.61 - 9.21 | m-vs |
| 15.9 - 16.3 | 5.57 - 5.44 | vw-m |
| 20.5 - 21.0 | 4.33 - 4.23 | m-vs |
| 24.3 - 25.1 | 3.66 - 3.548 | w-m |
| 30.5 - 31.1 | 2.931 - 2.876 | vw-m |

TABLE M (SENAPSO-46)

| 2θ | d (Å) | Relative Intensity |
|---|---|---|
| 7.2 - 8.1 | 12.28 - 10.92 | vs |
| 12.9 - 13.6 | 6.86 - 6.51 | vw |
| 21.2 - 22.2 | 4.19 - 4.501 | vw-m |
| 22.5 - 23.45 | 3.95 - 3.793 | vw-m |
| 26.6 - 27.9 | 3.351 - 3.198 | vw-m |

TABLE N (SENAPSO-47)

| 2θ | d (Å) | Relative Intensity |
|---|---|---|
| 9.4 - 9.6 | 9.41 - 9.21 | vs |
| 12.8 - 13.1 | 6.92 - 6.76 | vw-m |
| 16.0 - 16.3 | 5.54 - 5.44 | vw-m |
| 20.5 - 21.0 | 4.31 - 4.23 | m-vs |
| 24.6 - 25.3 | 3.613 - 3.526 | vm-m |
| 30.6 - 31.1 | 2.921 - 2.876 | vw-m |

The following examples are provided to further illustrate the invention and are not intended to be limiting thereof:

In the following examples the CoMnAPSO compositions were prepared using numerous reagents. The reagents employed and abbreviations employed herein, if any, for such reagents are as follows:

a) Aiipro: aluminum isopropoxide;

b) LUDOX—LS: LUDOX—LS is the trade name of DuPont from an aqueous solution of 30 weight percent $SiO_2$ and 0.1 weight percent $Na_2O$;

c) $H_3PO_4$: 85 weight percent phosphoric acid;

d) MnAc: Manganese acetate, $Mn(C_2H_3O_2)_2 \cdot 4H_2O$;

e) CoAc: Cobalt Acetate, $Co(C_2H_3O_2)_2 \cdot 4H_2O$;

f) TEAOH: 40 weight percent aqueous solution of tetraethylammonium hydroxide; and

g) $Pr_2NH$: di-n-propylamine, $(C_3H_7)_2NH$.

The following preparative examples were carried out by forming a starting reaction mixture by adding the $H_3PO_4$ and one half of the quantity of water. To this mixture the aluminum isopropoxide was added. This mixture was then blended until a homogeneous mixture was observed. To this mixture the LUDOX—LS was added and the resulting mixture blended (about 2 minutes) until a homogeneous mixture was observed. A second mixture was prepared using manganese acetate and one half of the remaining water. A third mixture was prepared using cobalt acetate and one half of the remaining water. The three mixtures were admixed were admixed and the resulting mixture blended until a homogeneous mixture was observed. The organic templating agent was then added to the resulting mixture and the resulting mixture blended until a homogeneous mixture was observed, i.e. about 2 to 4 minutes. The pH of the mixture was measured and adjusted for temperature. The mixture was then placed in a lined (polytetrafluoroethylene) stainless steel pressure vessel and digested at a temperature. All digestions were carried out at the autogeneous pressure.

## Examples 1 to 4

CoMnAPSO molecular sieves were prepared according to the above identified procedure and the CoMnAPSO products determined by X-ray analysis. The results of example 1 to 4 are set forth in Table I. Examples A to F in Table I represent reaction mixtures that did not show CoMnAPSO products when determined by X-ray analysis.

TABLE I

| Example[1] | Template | Temp (°C) | Time (days) | CoMnMqAPSO Products(s)[2] |
|---|---|---|---|---|
| 1 | TEAOH | 150 | 2 | CoMnAPSO—34; CoMnAPSO—5 |
| 2 | TEAOH | 150 | 7 | CoMnAPSO—34; CoMnAPSO—5 |
| 3 | $Pr_2NH$ | 200 | 2 | CoMnAPSO—5; CoMnAPSO—11 |
| 4 | $Pr_2NH$ | 200 | 7 | CoMnAPSO—5; CoMnAPSO—11 |
| A | TEAOH | 100 | 3 | — |
| B | TEAOH | 100 | 7 | — |
| C | $Pr_2NH$ | 150 | 2 | — |
| D[3] | $Pr_2NH$ | 150 | 10 | — |
| E[3] | $Pr_2NH$ | 150 | 6 | — |
| F[3] | $Pr_2NH$ | 150 | 15 | — |

[1]. The reaction mixture comprises:

$1.0R:0.2 MnO:0.2 CoO:0.8 Al_2O_3:0.8 P_2O_5:0.4 SiO_2:50 H_2O$

where "R" is the template.

[2]. Major species as identified by X-ray powder diffraction pattern of product, except that when two species are identified the first species listed is present in an amount equal to or greater than the second species listed. A "-" indicates that crystalline products were not identified by X-ray analysis.

[3]. X-ray analysis indicated that crystalline product was beginning to form.

Example 5

(a) Samples of the above prepared CoMnAPSO products, as identified in parenthesis, were calcined in air to remove at least part of the organic templating agent of the CoMnAPSO product. The adsorption capacities of each calcined sample were measured using a standard McBain-Bakr gravimetric adsorption apparatus. The samples were activated in a vacuum (less than 0.04 torr (0.053 mbar) at 350°C prior to measurement. The McBain-Bakr data were as follows:

(b) CoMnAPSO—34 and CoMnAPSO—5 (Example 2):

| Adsorbate | Kinetic Diameter, Å | Pressure (Torr)(x 1.333 mbar) | Temp (°C) | Wt.% Adsorbed* |
|---|---|---|---|---|
| Oxygen | 3.46 | 105 | -183 | 13.8 |
| Oxygen | 3.46 | 733 | -183 | 18.5 |
| Neopentane | 6.2 | 742 | 23.8 | 2.6 |
| Cyclohexane | 6.0 | 65 | 23.7 | 4.6 |
| n-hexane | 4.3 | 93 | 23.4 | 5.0 |
| $H_2O$ | 2.65 | 4.6 | 23.4 | 15.8 |
| $H_2O$ | 2.65 | 19 | 23.7 | 23.6 |

*calcined in air at 600°C for one hour prior to activation

(c) CoMnAPSO-5 and CoMnAPSO-11 (Example 4):

| Adsorbate | Kinetic Diameter, Å | Pressure (Torr)(x 1.333 mbar) | Temp (°C) | Wt.% Adsorbed* |
|---|---|---|---|---|
| Oxygen | 3.46 | 105 | -183 | 5.5 |
| Oxygen | 3.46 | 733 | -183 | 9.3 |
| Neopentane | 6.2 | 742 | 23.8 | 2.4 |
| Cyclohexane | 6.0 | 65 | 23.7 | 5.9 |
| $H_2O$ | 2.65 | 4.6 | 23.4 | 7.4 |
| $H_2O$ | 2.65 | 19 | 23.7 | 16.2 |

*calcined in air at 600°C for one hour prior to activation

Example 6

Samples of the as-synthesized products of examples 2 and 4 were subjected to chemical analysis. The chemical analysis for these CoMnAPSOs was:

(a) The chemical analysis for the product of example 2 was:

| Component | Weight Percent |
|---|---|
| $Al_2O_3$ | 27.5 |
| $P_2O_5$ | 37.7 |
| $SiO_2$ | 4.98 |
| CoO | 4.3 |
| MnO | 5.2 |
| Carbon | 5.3 |
| LOI* | 20.5 |

*Loss on Ignition

The above chemical analysis gives an overall product composition in molar oxide ratios (anhydrous basis) of:

$0.057\ CoO:0.073\ MnO:0.270\ Al_2O_3:0.266\ P_2O_5:0.083\ SiO_2$

and a formula (anhydrous basis) of:

$0.055R\ (Al_{0.420}P_{0.414}Si_{0.065}Co_{0.044}Mn_{0.057})O_2$

(b) The chemical analysis for the product of example 4 was:

| Component | Weight Percent |
|---|---|
| $Al_2O_3$ | 26.6 |
| $P_2O_5$ | 37.6 |
| $SiO_2$ | 7.1 |
| CoO | 5.1 |
| MnO | 6.0 |
| Carbon | 1.91 |
| LOI* | 17.9 |

*Loss on Ignition

The above chemical analysis gives an overall product composition in molar oxide ratios (anhydrous basis) of:

$0.068\ CoO:0.085\ MnO:0.261\ Al_2O_3:0.265\ P_2O_5:0.118\ SiO_2$

and a formula (anhydrous basis of:

$0.027R\ (Al_{0.40}P_{0.40}Si_{0.089}Co_{0.051}Mn_{0.064})O_2$

Example 7

EDAX (energy dispersive analysis by X-ray microprobe analysis in conjunction with SEM (scanning electron microscope) was carried out on the products of examples 2 and 4. Analysis of crystals having a morphology characteristic of each CoMnAPSO product gave the following analysis based on relative peak heights:

(a) Example 2 (CoMn APSO—5):

| | Average of Spot Probes |
|---|---|
| Al | 0.81 |
| P | 0.98 |
| Si | 0.18 |
| Co | 0.10 |
| Mn | 0.17 |

(b) Example 2 (CoMnAPSO—34):

| | Average of Spot Probes |
|---|---|
| Al | 0.82 |
| P | 0.93 |
| Si | 0.17 |
| Co | 0.03 |
| Mn | 0.03 |

(c) Example 3 (CoMnAPSO—5):

| | Average of Spot Probes |
|---|---|
| Al | 0.93 |
| P | 0.71 |
| Si | 0.15 |
| Co | 0.05 |
| Mn | 0.07 |

(d) Example 4 (CoMnAPSO—11):

| | Average of Spot Probes |
|---|---|
| Al | 0.81 |
| P | 0.95 |
| Si | 0.15 |
| Co | 0.03 |
| Mn | 0.05 |

Example 8

(a) CoMnAPSO—5, as prepared in example 1, was subjected to X-ray analysis. The CoMnAPSO—5 was determined to have an X-ray powder diffraction pattern characterized by the following data:

| 2θ | d (Å) | (I/Io)x100 |
|---|---|---|
| 7.5 | 11.84 | 67 |
| 9.5* | 9.29 | 100 |
| 12.9** | 6.89 | 11 |
| 14.1* | 6.29 | 7 |
| 14.9 | 5.93 | 14 |
| 16.0* | 5.54 | 22 |
| 18.0* | 4.93 | 10 |
| 19.8 | 4.49 | 19 |
| 20.6* | 4.32 | 51 |
| 21.1** | 4.22 | 40 |
| 22.4 | 3.96 | 28 |
| 25.2* | 3.530 | 12 |
| 29.1 | 3.071 | 6 |
| 29.5* | 3.024 | 3 |
| 30.1 | 2.968 | 10 |
| 30.5* | 2.928 | 16 |
| 31.3* | 2.862 | 11 |
| 33.7* | 2.659 | 3 |
| 34.5 | 2.601 | 4 |
| 34.6* | 2.591 | 5 |
| 37.8 | 2.383 | 6 |
| 47.7** | 1.905 | 3 |
| 48.9* | 1.863 | 2 |
| 49.9* | 1.828 | 2 |
| 50.9* | 1.794 | 2 |
| 55.8 | 1.647 | 2 |

*peak may be an impurity

**impurity peak and CoMnMgAPSO-5

(b) A portion of the as-synthesized CoMnAPSO—5 of example 2 was calcined in air at 600°C for one (1) hour. The calcined products was characterized by the following X-ray powder diffraction pattern:

| 2θ | d (Å) | (I/Io)x100 |
|---|---|---|
| 7.5 | 11.84 | 32 |
| 9.6* | 9.20 | 100 |
| 13.0** | 6.81 | 20 |
| 14.9 | 5.93 | 4 |
| 16.2* | 5.48 | 8 |
| 18.0* | 4.93 | 6 |
| 19.3* | 4.60 | 3 |
| 19.8 | 4.49 | 8 |
| 20.9** | 4.26 | 22 |
| 21.2** | 4.20 | 26 |
| 21.5* | 4.13 | 3 |
| 22.5 | 3.95 | 32 |
| 23.4* | 3.81 | 3 |
| 25.3* | 3.520 | 7 |
| 26.1 | 3.420 | 11 |
| 26.2* | 3.396 | 7 |
| 28.5* | 3.129 | 3 |
| 29.2 | 3.063 | 6 |
| 30.2 | 2.965 | 6 |
| 31.0* | 2.881 | 11 |
| 31.5* | 2.840 | 7 |
| 34.7 | 2.584 | 4 |
| 34.9 | 2.568 | 3 |
| 38.0* | 2.368 | 2 |

*peak may be an impurity

**impurity peak and CoMnAPSO-5

(c) The species CoMnAPSO—5 is a molecular sieve having a characteristic X-ray powder diffraction pattern which contains at least the d-spacings set forth in Table II as follows

Table II

| 2θ | d (Å) | Relative Intensity |
|---|---|---|
| 7.4-7.5 | 11.95-11.84 | m |
| 12.9-13.1 | 6.89-6.76 | w-m |
| 14.9 | 5.93 | vw-w |
| 19.7-19.8 | 4.51-4.49 | vw-w |
| 20.9-21.3 | 4.26-4.17 | m |
| 22.4-22.5 | 3.97-3.95 | m |

(d) All of the CoMnAPSO—5 compositions, both as-synthesized and calcined, for which X-ray powder diffraction data have been obtained have patterns which are within the generalized pattern of Table III, below:

Table III

| 2θ | d (Å) | (I/Io)x100 |
|---|---|---|
| 7.4 - 7.5 | 11.95 - 11.84 | 32 - 67 |
| 12.9 - 13.1 | 6.89 - 6.81 | 11 - 20 |
| 14.9 | 5.93 | 4 - 14 |
| 19.7 - 19.8 | 4.51 - 4.49 | 8 - 19 |
| 20.9 - 21.3 | 4.26 - 4.17 | 22 - 40 |
| 22.4 - 22.5 | 3.96 - 3.95 | 28 - 32 |
| 24.7 - 24.8 | 3.60 - 3.59 | 6 |
| 25.9 - 26.1 | 3.440 - 3.420 | 10 - 11 |
| 29.0 - 29.2 | 3.079 - 3.063 | 6 |
| 29.9 - 30.2 | 2.988 - 2.965 | 6 - 10 |
| 34.4 - 34.7 | 2.607 - 2.584 | 4 |
| 34.9 | 2.568 | 3 |
| 37.8 | 2.383 | 6 |
| 47.7 | 1.905 | 3 |
| 55.8 | 1.647 | 2 |

Example 9

(a) The CoMnAPSO—11, prepared in example 3, was subjected to X-ray analysis. The CoMnAPSO—11 was impure but the CoMnAPSO—11 was determined to have an X-ray powder diffraction pattern characterized by the following data;

| 2θ | d (Å) | (I/Io)x100 |
|---|---|---|
| 7.0* | 12.56 | 12 |
| 7.5* | 11.86 | 68 |
| 8.1 | 10.88 | 46 |
| 9.5 | 9.31 | 68 |
| 12.9* | 6.87 | 11 |
| 13.2 | 6.73 | 24 |
| 14.9* | 5.95 | 12 |
| 15.7 | 5.64 | 49 |
| 16.3 | 5.44 | 9 |
| 19.0 | 4.67 | 9 |
| 19.7* | 4.50 | 29 |
| 20.4 | 4.36 | 66 |
| 21.1** | 4.21 | 37 |
| 21.2 | 4.19 | 34 |
| 22.4* | 3.96 | 41 |
| 22.8 | 3.91 | 29 |
| 23.2 | 3.83 | 100 |

| 2θ | d (Å) | (I/Io)x100 |
|---|---|---|
| 24.8** | 3.59 | 10 |
| 25.9* | 3.443 | 23 |
| 26.5 | 3.365 | 32 |
| 28.2 | 3.163 | 9 |
| 28.7 | 3.113 | 25 |
| 29.5 | 3.024 | 8 |
| 29.9* | 2.985 | 15 |
| 31.5 | 2.838 | 8 |
| 32.7 | 2.739 | 2 |
| 34.2 | 2.622 | 2 |
| 36.4 | 2.468 | 2 |
| 37.6 | 2.392 | 2 |

* peak may be an impurity
**impurity peak

(b) A portion of the as-synthesized CoMnAPSO—11 of example 4 was calcined in air at 600°C for one (1) hour. The calcined product was characterized by the following X-ray powder diffraction pattern:

| 2θ | d (Å) | (I/Io)x100 |
|---|---|---|
| 7.5* | 11.86 | 95 |
| 8.2 | 10.85 | 68 |
| 9.6 | 9.19 | 95 |
| 13.1* | 6.77 | 45 |
| 15.9 | 5.58 | 91 |
| 19.8* | 4.48 | 32 |
| 20.3 | 4.37 | 49 |
| 21.3* | 4.17 | 34 |
| 22.5** | 3.96 | 62 |
| 23.4 | 3.80 | 100 |
| 26.0* | 3.423 | 43 |
| 26.4 | 3.376 | 40 |
| 26.6 | 3.346 | 16 |
| 29.1* | 3.073 | 27 |
| 29.2 | 3.061 | 28 |
| 30.2* | 2.962 | 21 |
| 32.8 | 2.732 | 21 |
| 32.9 | 2.719 | 31 |
| 34.7* | 2.586 | 28 |
| 36.2 | 2.481 | 2 |

*peak may contain impurity
**impurity peak

(c) The species CoMnAPSO—11 is a molecular sieve having a characteristic X-ray powder diffraction pattern which contains at least the d-spacings set forth in Table IV as follows:

Table IV

| 2θ | d (Å) | Relative Intensity |
|---|---|---|
| 9.5-9.6 | 9.31-9.21 | m-vs |
| 15.7-15.9 | 5.64-5.57 | m-vs |
| 20.3-20.4 | 4.37-4.36 | m |
| 21.1-21.2 | 4.21-4.19 | m |
| 22.1-22.5 | 4.02-3.95 | m |
| 23.2-23.4 | 3.83-3.80 | vs |

(d) All of the CoMnAPSO—11 compositions, both as-synthesized and calcined, for which X-ray powder diffraction data have presently been obtained have patterns which are within the generalized pattern of Table V, below:

Table V

| 2θ | d (Å) | (I/Io)x100 |
|---|---|---|
| 8.1-8.2 | 10.88-10.85 | 46-68 |
| 9.5-9.6 | 9.31-9.19 | 68-95 |
| 13.1-13.2 | 6.77-6.73 | 24-45 |
| 15.7-15.9 | 5.64-5.58 | 49-91 |
| 16.3 | 5.44 | 9 |
| 19.0 | 4.67 | 9-10 |
| 20.3-20.4 | 4.37-4.36 | 49-66 |
| 21.1-21.2 | 4.21-4.19 | 30-37 |
| 22.1-22.5 | 4.02-3.96 | 31-62 |
| 22.7-22.8 | 3.92-3.91 | 28-29 |
| 23.2-23.4 | 3.83-3.80 | 100 |
| 24.7-24.8 | 3.60-3.59 | 10-14 |
| 26.4-26.6 | 3.376-3.346 | 16-40 |
| 28.1-28.2 | 3.175-3.163 | 9 |
| 28.7 | 3.113 | 25-26 |
| 29.2-29.5 | 3.061-3.024 | 8-28 |
| 31.5 | 2.838 | 8 |
| 32.7-32.8 | 2.739-2.732 | 2-27 |
| 32.9 | 2.719 | 31 |
| 34.2 | 2.622 | 2-11 |
| 36.2-36.4 | 2.481-2.468 | 2-9 |
| 37.6-37.9 | 2.392-2.374 | 2-3 |

Example 10

(a) The CoMnAPSO—34, prepared in example 1, was subjected to X-ray analysis. The CoMnAPSO—34 was impure but was the major phase and was determined to have an X-ray powder diffraction pattern characterized by the following data:

| 2θ | d (Å) | (I/Io)x100 |
|---|---|---|
| 7.5* | 11.84 | 67 |
| 9.5 | 9.29 | 100 |
| 12.9** | 6.89 | 11 |
| 14.1 | 6.29 | 7 |
| 14.9* | 5.93 | 14 |
| 16.0 | 5.54 | 22 |
| 18.0 | 4.93 | 10 |
| 19.8* | 4.49 | 19 |
| 20.6 | 4.32 | 51 |
| 21.1** | 4.22 | 40 |
| 22.4* | 3.96 | 28 |
| 25.2 | 3.530 | 12 |
| 29.1* | 3.071 | 6 |
| 29.5 | 3.024 | 3 |
| 30.1* | 2.968 | 10 |
| 30.5 | 2.928 | 16 |
| 31.3 | 2.862 | 11 |
| 33.7 | 2.659 | 3 |
| 34.5* | 2.601 | 4 |
| 34.6 | 2.591 | 5 |
| 37.8* | 2.383 | 6 |
| 47.7** | 1.905 | 3 |
| 48.9 | 1.863 | 2 |
| 49.9 | 1.828 | 2 |
| 50.9 | 1.794 | 2 |
| 55.8* | 1.647 | 2 |

*peak may contain impurity

**impurity peak

(b) A portion of the as-synthesized CoMnAPSO—34 of 2 was calcined in air at 600°C for one (1) hour. The calcined products was characterized by the following X-ray powder diffraction pattern:

| 2θ | d (Å) | (I/Io)x100 |
|---|---|---|
| 7.5* | 11.84 | 32 |
| 9.6 | 9.20 | 100 |
| 13.0** | 6.81 | 20 |
| 14.9* | 5.93 | 4 |
| 16.2 | 5.48 | 8 |
| 18.0 | 4.93 | 6 |
| 19.3 | 4.60 | 3 |
| 19.8* | 4.49 | 8 |
| 20.9** | 4.26 | 22 |
| 21.2** | 4.20 | 26 |
| 21.5 | 4.13 | 3 |
| 22.5* | 3.96 | 32 |
| 23.4 | 3.81 | 3 |
| 25.3 | 3.520 | 7 |
| 26.1* | 3.420 | 11 |
| 26.2 | 3.396 | 7 |
| 28.5 | 3.129 | 3 |
| 29.2* | 3.063 | 6 |
| 30.2* | 2.965 | 6 |
| 31.0 | 2.881 | 11 |
| 31.5 | 2.840 | 7 |
| 34.7* | 2.584 | 4 |
| 34.9* | 2.568 | 3 |
| 38.0 | 2.368 | 2 |

*peak may contain impurity

**impurity peak

(c) The species CoMnAPSO—34 is a molecular sieve having a characteristic X-ray powder diffraction pattern which contains at least the d-spacings set forth in Table VI as follows:

Table VI

| 2θ | d (Å) | Relative Intensity |
|---|---|---|
| 9.5-9.6 | 9.29-9.20 | vs |
| 12.8-13.0 | 6.92-6.81 | w-m |
| 16.0-16.2 | 5.54-5.48 | vw-m |
| 20.6-20.9 | 4.32-4.26 | m |
| 21.1-21.2 | 4.22-4.20 | m |
| 25.2-25.3 | 3.530-3.520 | vw-w |
| 31.0-31.5 | 2.881-2.840 | w |

(d) All of the CoMnAPSO—34 compositions, both as-synthesized and calcined, for which X-ray powder diffraction data have been obtained have patterns which are within the generalized pattern below:

Table VII

| 2θ | d (Å) | (I/Io)x100 |
|---|---|---|
| 9.5-9.6 | 9.29-9.20 | 100 |
| 12.8-13.0 | 6.92-6.81 | 11-20 |
| 14.1 | 6.29 | 7-9 |
| 16.0-16.2 | 5.54-5.48 | 8-23 |
| 18.0 | 4.93 | 6-12 |
| 19.3 | 4.60 | 3 |
| 20.6-20.9 | 4.32-4.26 | 22-57 |
| 21.1-21.2 | 4.22-4.20 | 26-40 |
| 21.5 | 4.13 | 3 |
| 23.0-23.4 | 3.87-3.81 | 2-3 |
| 25.2-25.3 | 3.530-3.520 | 7-14 |
| 25.8-26.2 | 3.453-3.396 | 7-13 |
| 27.5 | 3.243 | 2 |
| 28.3-28.5 | 3.153-3.129 | 3-4 |
| 29.5 | 3.024 | 3 |
| 30.5 | 2.928 | 16-18 |
| 31.0-31.5 | 2.881-2.840 | 11-13 |
| 33.7-33.8 | 2.659-2.652 | 2-7 |
| 34.5-34.6 | 2.601-2.592 | 5 |
| 38.0 | 2.368 | 2 |
| 39.6 | 2.276 | 2 |
| 43.3 | 2.090 | 2 |
| 47.5-47.7 | 1.914-1.905 | 2-3 |
| 48.9-49.0 | 1.863-1.859 | 2-4 |
| 49.9 | 1.828 | 2 |
| 50.8-50.9 | 1.797-1.794 | 2-3 |

Preparative Reagents for Examples

In the following examples 11 to 20 the CoMnMgAPSO compositions were prepared using numerous reagents. The reagents employed and abbreviations employed herein, if any, for such reagents are as follows:

a) Alipro; aluminum isopropoxide;

b) LUDOX—LS: LUDOX—LS is the tradename of Du Pont for an aqueous solution of 30 weight percent $SiO_2$ and 0.1 weight percent $Na_2O$;

c) $H_3PO_4$: aqueous solution which is 85 weight percent phosphoric acid;

d) MnAc: Manganese acetate, $Mn(C_2H_3O_2)_2 \cdot 4H_2O$;

e) CpAc: Cobalt Acetate, $Co(C_2H_3O_2)_2 \cdot 4H_2O$;

f) MgAc: Magnesium Acetate $Mg(C_2H_3O_2) \cdot 4H_2O$;

g) TEAOH: 40 weight percent aqueous solution of tetraethylammonium hydroxide; and

h) $Pr_2NH$: di-n-propylamine, $(C_3H_7)_2NH$.

Preparative Procedures

The following preparative examples were carried out by forming a starting reaction mixture by adding the $H_3PO_4$ and one half of the quantity of water. To this mixture the aluminium isoproxide was added. This mixture was then blended until a homogeneous mixture was observed. To this mixture the LUDOX—LS was added and the resulting mixture blended (about 2 minutes) until a homogeneous mixture was observed.

Three additional mixtures were prepared using cobalt acetate, magnesium acetate and manganese acetate using one third of the remainder of the water for each mixture. The four mixtures were then admixed and the resulting mixture blended until a homogeneous mixture was observed. The organic templating agent was then added to the resulting mixture and the resulting mixture blended until a homogeneous mixture was observed, i.e., about 2 to 4 minutes. The mixture was then placed in a lined (polytetrafluoroethylene) stainless steel pressure vessel and digested at a temperature for a time. All digestions were carried out at the autogeneous pressure.

The molar composition for each preparation will be given by the relative moles of the components with $H_3PO_4$ be given as $P_2O_5$.

Examples 11 to 14

CoMnMgAPSO molecular sieves were prepared according to the identified procedure and the CoMnMgAPSO products determined by X-ray analysis. The results of preparative examples 11 to 14 are set forth in Table I—1. Examples A—1, B—1 and C—1 of Table I—1 did contain a CoMnMgAPSO product identifiable by X-ray analysis.

TABLE I—1

| Example[1] | Template | Temp (°C) | Time (days) | CoMnMgAPSO Product(s)[2] |
|---|---|---|---|---|
| 11 | TEAOH | 100 | 7 | CoMnMgAPSO—34 |
| 12 | TEAOH | 150 | 2 | CoMnMgAPSO—34; CoMnMgAPSO—5 |
| 13 | TEAOH | 150 | 7 | CoMnMgAPSO—34; CoMnMgAPSO—5 |
| 14 | $Pr_2NH$ | 200 | 13 | CoMnMgAPSO—11 |
| A—1 | TEAOH | 100 | 2 | " |
| B—1 | $Pr_2NH$ | 150 | 3 | " |
| C—1 | $Pr_2NH$ | 150 | 10 | " |

[1] Reaction mixture comprised:
1.0 R:0.2 MnO:0.2 CoO:0.2 MgO:0.7 $Al_2O_3$:0.8 $P_2O_5$:0.4 $SiO_2$:50 $H_2O$ where "R" is the template.

[2] Major species as identified by x-ray powder diffraction pattern of product, except that when two species are identified the species are listed in the order of their predominance in the product. A "—" indicates no SENAPSO product was identified by x-ray ananlysis.

Example 15

Portions of the products of examples 13 and 14 were calcined in air at 600°C for 1.5 hour to remove at least part of the organic templating agent. The adsorption capacities of each calcined sample were measured using a standard McBain-Baker gravimetric adsorption apparatus. The sample were activated in a vacuum (less than about 0.04 torr) at 350°C prior to measurement. The McBain-Baker data for the CoMnMgAPSO products were:

(a) Example 13: (CoMnMgAPSO-34 and CoMnMgAPSO-5)

| Adsorbate | Kinetic Diameter, Å | Pressure (Torr)(x 1.333 mbar) | Temp (°C) | Wt.% Adsorbed |
|---|---|---|---|---|
| Oxygen | 3.46 | 105 | -183 | 6.0 |
| Oxygen | 3.46 | 733 | -183 | 8.4 |
| Neopentane | 6.2 | 742 | 23.8 | 1.4 |
| Cyclohexane | 6.0 | 65 | 23.7 | 2.6 |
| n-hexane | 6.0 | 93 | 23.4 | 3.3 |
| $H_2O$ | 2.65 | 4.6 | 23.4 | 7.3 |
| $H_2O$ | 2.65 | 19 | 23.7 | 12.0 |

(b) Example 14: (CoMnMgAPSO-11)

| Adsorbate | Kinetic Diameter, Å | Pressure (Torr)(x 1.333 mbar) | Temp (°C) | Wt.% Adsorbed |
|---|---|---|---|---|
| Oxygen | 3.46 | 105 | -183 | 2.9 |
| Oxygen | 3.46 | 733 | -183 | 3.6 |
| Neopentane | 6.2 | 742 | 23.8 | 0.5 |
| Cyclohexane | 6.0 | 65 | 23.7 | 2.1 |
| $H_2O$ | 2.65 | 4.6 | 23.4 | 4.1 |
| $H_2O$ | 2.65 | 19 | 23.7 | 9.1 |

Example 16

Portions of the products of examples 13 and 14 were subjected to chemical analysis. The chemical analyses were as follows:

(a) Example 13:

| Component | Weight Percent |
|---|---|
| $Al_2O_3$ | 21.5 |
| $P_2O_5$ | 40.3 |
| $SiO_2$ | 6.5 |
| CoO | 4.58 |
| MnO | 4.41 |
| MgO | 2.43 |
| Carbon | 6.9 |
| LOI* | 18.3 |

*Loss on Ignition

The above chemical analysis gives an overall product composition in molar oxide ratios (anhydrous basis) of:

0.575 R:0.061 CoO:0.062 MnO:0.060 MgO:0.211 $Al_2O_3$:0.284 $P_2O_5$:0.108 $SiO_2$ and a formula (anhydrous basis) of:

0.072 R($Co_{0.048}Mn_{0.048}Mg_{0.047}Al_{0.33}P_{0.44}Si_{0.084}$)$O_2$.

(b) Example 14:

| Component | Weight Percent |
|---|---|
| $Al_2O_3$ | 24.3 |
| $P_2O_5$ | 41.8 |
| $SiO_2$ | 8.5 |
| CoO | 6.0 |
| MnO | 6.8 |
| MgO | 2.8 |

| Component | Weight Percent |
|---|---|
| Carbon | 1.54 |
| LOI* | 9.3 |

*Loss on Ignition

The above chemical analysis gives an overall product composition in molar oxidce ratios (anhydrous basis) of:

$$0.128\ R:0.08\ CoO:0.096\ MnO:0.070\ MgO:0.238\ Al_2O_3:0.294\ P_2O_5:0.141\ SiO_2$$

and a formula (anhydrous basis) of:

$$0.0213\ R(Co_{0.055}\ Mn_{0.066}\ Mg_{0.048}\ Al_{0.33}\ P_{0.41}\ Si_{0.09})O_2$$

Example 17

EDAX (energy analysis by x-ray) microprobe analysis in conjunction with SEM (scanning electron microscope) was carried out on clean crystals of products from examples 13 and 14. Analysis of crystals having a morphology characteristic of CoMnMgAPSO—5, CoMnMgAPSO—11, and CoMnMgAPSO—34 gave the following analysis based on relative peak heights:

(a) CoMnMgAPSO-5:

| | Average of Spot Probes |
|---|---|
| Co | 0.11 |
| Mn | 0.16 |
| Mg | 0.08 |
| Al | 0.55 |
| P | 1.0 |
| Si | 0.11 |

(b) CoMnMgAPSO-11:

| | Average of Spot Probes |
|---|---|
| Co | 0.09 |
| Mn | 0.06 |
| Mg | 0.11 |
| Al | 0.85 |
| P | 0.99 |
| Si | 0.38 |

(c) CoMnMgAPSO-34:

| | Average of Spot Probes |
|---|---|
| Co | 0.05 |
| Mn | 0.03 |
| Mg | 0.05 |
| Al | 0.81 |
| P | 1.0 |
| Si | 0.20 |

Example 18

(a) CoMnMgAPSO—5, as prepared to in example 13, was subjected to x-ray analysis, and was determined to have a characteristic x-ray powder diffraction pattern which contains the d-spacings set forth below:

| 2θ | d (Å) | 100x I/Io |
|---|---|---|
| 7.4 | 11.89 | 15 |
| 9.5* | 9.27 | 100 |
| 12.8** | 6.90 | 19 |
| 14.1* | 6.28 | 14 |
| 14.9 | 5.96 | 6 |
| 16.0* | 5.54 | 46 |
| 18.1* | 4.90 | 28 |
| 19.2* | 4.63 | 12 |
| 19.7 | 4.50 | 16 |
| 20.6* | 4.32 | 92 |
| 21.1 | 4.20 | 13 |
| 22.4* | 3.97 | 22 |
| 22.6 | 3.94 | 5 |
| 23.1* | 3.85 | 6 |
| 25.2* | 3.529 | 28 |
| 25.8** | 3.454 | 32 |
| 27.6* | 3.237 | 4 |
| 28.4* | 3.142 | 4 |
| 29.0 | 3.079 | 5 |
| 29.5* | 3.025 | 4 |
| 29.9 | 2.987 | 7 |
| 30.5** | 2.930 | 37 |
| 31.3* | 2.863 | 25 |
| 32.4* | 2.767 | 26 |
| 34.4** | 2.608 | 11 |
| 35.4* | 2.537 | 5 |
| 36.3* | 2.473 | 5 |
| 37.8 | 2.382 | 4 |

| 2θ | d (Å) | 100x I/Io |
|---|---|---|
| 38.7* | 2.329 | 6 |
| 38.8* | 2.323 | 6 |
| 39.6* | 2.276 | 5 |
| 43.3* | 2.088 | 5 |
| 45.1 | 2.010 | 3 |
| 46.1* | 1.971 | 4 |
| 46.3 | 1.962 | 5 |
| 47.2* | 1.924 | 7 |
| 48.7 | 1.870 | 6 |
| 48.9* | 1.863 | 6 |
| 51.0* | 1.791 | 4 |
| 53.0* | 1.728 | 4 |
| 53.1* | 1.726 | 4 |

*peak may be an impurity
**impurity peak and CoMnMgAPSO-5

(b) A portion of the as-synthesized CoMnMgAPSO—5 of part (a) was calcined in air at 600°C for one (1) hour. The calcined product was characterized by the x-ray powder diffraction pattern below:

| 2θ | d, (Å) | 100xI/Io |
|---|---|---|
| 7.5 | 11.76 | 100 |
| 9.7* | 9.14 | 86 |
| 13.1** | 6.79 | 18 |
| 15.0 | 5.90 | 30 |
| 16.3* | 5.44 | 8 |
| 18.1* | 4.90 | 7 |
| 19.9 | 4.47 | 19 |
| 21.2** | 4.19 | 35 |
| 21.5* | 4.13 | 44 |
| 22.6 | 3.94 | 37 |
| 23.0* | 3.87 | 6 |
| 26.1 | 3.414 | 21 |
| 26.4* | 3.379 | 9 |
| 29.2 | 3.060 | 8 |
| 30.2 | 2.956 | 59 |
| 31.2* | 2.871 | 12 |
| 31.7* | 2.819 | 7 |
| 34.7 | 2.582 | 13 |
| 35.5* | 2.528 | 16 |

*peak may be an impurity
**impurity peak and CoMnMgAPSO-5

(c) The species CoMnMgAPSO—5 is a molecular sieve having a three-dimensional microporous framework structure of $CoO_2^{-2}$, $MnO_2^{-2}$, $MgO_2^{-2}$, $AlO_2^-$, $PO_2^+$, and $SiO_2$ tetrahedral oxide units and have an empirical chemical composition on an anhydrous basis expressed by the formula:

$$mR:(Co_tMn_uMg_vAl_xP_ySi_z)O_2$$

wherein "R" represents an organic templating agent present in the intracrystalline pore system; "m" represents the molar amount of "R" present per mole of $(Co_tMn_uMg_vAl_xP_ySi_z)O_2$ and has a value of from zero to about 0.3; and "t", "u", "v", "x", "y", and "z", where "w" is the sum of "t + u + v", represent the mole fractions of cobalt, manganese, magnesium, aluminum, phosphorus and silicon, respectively, present as tetrahedral oxides, said mole fractions being within the compositional area defined by points A, B, C, D and E of Fig. 1, more preferably by the compositional area defined by points a, b, c, d, e and f of Fig. 2, and having a characteristic x-ray powder diffraction pattern which contains at least the d-spacings set forth below:

| 2θ | d, (Å) | Relative Intensity |
|---|---|---|
| 7.2-7.7 | 12.28-11.48 | m-vs |
| 19.4-19.9 | 4.58-4.46 | w-m |
| 20.85-21.3 | 4.26-4.17 | w-vs |
| 22.1-22.6 | 4.02-3.93 | m-vs |
| 25.6-26.1 | 3.480-3.414 | vw-m |

(d) The CoMnMgAPSO—5 compositions for which x-ray powder diffraction data have been obtained have patterns which are characterized by the data set forth below:

Table III

| 2θ | d, (Å) | 100xI/Io |
|---|---|---|
| 7.4-7.5 | 11.89-11.76 | 15-100.0 |
| 12.8-13.1 | 6.90-6.79 | 16-19 |
| 14.9-15.0 | 5.96-5.90 | 6-30 |
| 19.7-19.9 | 4.50-4.47 | 16-19 |
| 21.1-21.2 | 4.20-4.19 | 10-35 |
| 22.6 | 3.94 | 5-37 |
| 25.8-26.1 | 3.454-3.414 | 18-32 |
| 29.0-29.2 | 3.079-3.060 | 4-8 |
| 29.9-30.2 | 2.987-2.956 | 7-59 |
| 30.5 | 2.930 | 28-37 |
| 34.4-34.7 | 2.608-2.582 | 11-14 |
| 37.8 | 2.382 | 4 |
| 45.1 | 2,010 | 3 |
| 46.3 | 1.962 | 5 |
| 48.7 | 1.870 | 6 |

Example 19

(a) CoMnMgAPSO—11, as prepared in example 16 was subjected to x-ray analysis. CoMnMgAPSO—11 was determined to have a characteristic x-ray powder diffraction pattern which contains the d-spacings set forth below:

| 2θ | d, (Å) | 100xI/Io |
|---|---|---|
| 8.1 | 10.95 | 21 |
| 9.5 | 9.35 | 34 |
| 13.1 | 6.75 | 9 |
| 15.7 | 5.66 | 22 |
| 20.3 | 4.37 | 29 |
| 21.1 | 4.21 | 75 |
| 22.1 | 4.02 | 34 |
| 22.4 | 3.97 | 27 |
| 22.7 | 3.92 | 34 |
| 23.1 | 3.84 | 53 |
| 24.7 | 3.61 | 7 |
| 26.4 | 3.374 | 23 |
| 27.6* | 3.234 | 100 |
| 28.6 | 3.124 | 75 |
| 32.7 | 2.736 | 13 |
| 35.2 | 2.548 | 20 |
| 37.5 | 2.396 | 8 |
| 37.8 | 2.383 | 9 |
| 37.9 | 2.373 | 7 |
| 40.1 | 2.247 | 12 |
| 45.0 | 2.013 | 11 |
| 45.2 | 2.006 | 18 |
| 45.3 | 2.001 | 20 |
| 45.8 | 1.983 | 13 |
| 45.9 | 1.977 | 13 |
| 50.4 | 1.812 | 10 |
| 50.6 | 1.803 | 15 |

*peak may contain an impurity

(b) A portion of the as-synthesized of part (a) was calcined in air at 600°C for one (1) hour. The calcined product was characterized by the x-ray powder diffraction below:

| 2θ | d, (Å) | 100xI/Io |
|---|---|---|
| 8.1 | 10.95 | 31 |
| 9.6 | 9.23 | 43 |
| 13.0 | 6.80 | 30 |
| 15.8 | 5.60 | 37 |
| 20.2 | 4.40 | 27 |
| 21.3 | 4.18 | 100 |
| 22.3 | 3.99 | 65 |
| 23.0 | 3.87 | 36 |
| 23.4 | 3.80 | 50 |
| 24.4 | 3.65 | 11 |
| 26.3 | 3.392 | 25 |
| 28.3 | 3.157 | 83 |
| 28.9 | 3.090 | 17 |
| 29.1 | 3.067 | 11 |
| 32.8 | 2.734 | 19 |
| 34.3 | 2.614 | 12 |
| 37.9 | 2.373 | 12 |
| 39.0 | 2.309 | 15 |
| 39.3 | 2.294 | 14 |
| 44.8 | 2.025 | 16 |
| 44.9 | 2.021 | 17 |

(c) The species CoMnMgAPSO—11 is a molecular sieve having three-dimensional microporous framework structures of $CoO_2^{-2}$, $MnO_2^{-2}$, $MgO_2^{-2}$, $AlO_2^-$, $PO_2^+$, and $SiO_2$ tetrahedral oxide units and have an empirical chemical composition on an anhydrous basis expressed by the formula:

$$mR:(Co_tMn_uMg_vAl_xP_ySi_z)O_2$$

wherein "R" represents an organic templating agent present in the intracrystalline pore system; "m" represents the molar amount of "R" present per mole of $(Co_tMn_uMg_vAl_xP_ySi_z)O_2$ and has a value of from zero to about 0.3; and "t", "u", "v", "x", "y", and "z", where "w" is the sum of "t + u + v", represent the mole fractions of cobalt, manganese, magnesium, aluminum, phosphorus and silicon, respectively, present as tetrahedral oxides, said mole fractions being within the compositional area defined by points A, B, C, D and E of Fig. 1, more preferably by the compositional area defined by points a, b, c, d, e and f of Fig. 2, and having a characteristic x-ray powder diffraction pattern which contains at least the d-spacings set forth below:

| 2θ | d, (Å) | Relative Intensity |
|---|---|---|
| 7.8-8.2 | 11.19-10.85 | m-s |
| 9.0-9.8 | 9.83-9.03 | vw-vs |
| 12.8-13.6 | 6.92-6.51 | vw-m |
| 19.9-20.5 | 4.46-4.33 | m-s |
| 20.8-21.8 | 4.27-4.08 | m-vs |
| 22.0-22.6 | 4.04-3.93 | m-vs |
| 22.6-23.1 | 3.93-3.85 | vw-vs |
| 23.1-23.5 | 3.85-3.79 | w-vs |

(d) The CoMnMgAPSO—5 compositions for which x-ray powder diffraction data have been obtained have patterns which are characterized by the data set forth below:

| 2θ | d, (Å) | 100xI/Io |
|---|---|---|
| 8.1-8.2 | 10.95-10.65 | 17-31 |
| 9.5-9.6 | 9.35-9.23 | 34-46 |
| 13.0-13.3 | 6.80-6.66 | 9-30 |
| 15.7-15.8 | 5.66-5.60 | 22-37 |
| 20.2-20.4 | 4.40-4.35 | 27-29 |
| 21.1-21.4 | 4.21-4.15 | 75-100 |
| 22.1-22.3 | 4.02-3.99 | 34-65 |
| 22.4 | 3.97 | 27 |
| 22.7 | 3.92 | 34-100 |
| 23.0-23.2 | 3.87-3.83 | 36-53 |
| 23.3-23.4 | 3.82-3.80 | 50-70 |
| 24.4-24.7 | 3.65-3.61 | 7-11 |
| 26.3-26.5 | 3.392-3.363 | 23-25 |
| 28.3-28.7 | 3.157-3.110 | 75-83 |
| 28.9 | 3.090 | 16 |
| 29.1-30.4 | 3.067-2.940 | 11-14 |
| 32.7-32.8 | 2.739-2.734 | 13-19 |
| 34.3 | 2.614 | 12 |
| 35.2 | 2.548 | 20 |
| 37.5-37.8 | 2.398-2.383 | 8 |
| 37.9 | 2.373 | 7-12 |
| 39.0 | 2.309 | 15 |
| 39.3-40.1 | 2.294-2.247 | 12-16 |
| 44.8-45.0 | 2.025-2.013 | 11-17 |
| 45.2 | 2.006 | 18 |

| 2θ | d, (Å) | 100xI/Io |
|---|---|---|
| 45.3 | 2.001 | 20 |
| 45.8 | 1.983 | 13 |
| 45.9 | 1.977 | 13 |
| 50.4 | 1.812 | 10 |
| 50.6 | 1.803 | 15 |

Example 20

(a) CoMnMgAPSO—34, as prepared in example 13, was subjected to x-ray analysis. CoMnMgAPSO—34 was determined to have a characteristic x-ray powder diffraction pattern which contains at least the d-spacings set forth below:

| 2θ | d, (Å) | 100xI/Io |
|---|---|---|
| 7.4* | 11.89 | 15 |
| 9.5 | 9.31 | 100 |
| 12.8** | 6.90 | 19 |
| 14.1 | 6.28 | 14 |
| 14.9* | 5.96 | 6 |
| 16.0 | 5.54 | 46 |
| 18.1 | 4.90 | 28 |
| 19.2 | 4.62 | 12 |
| 19.7* | 4.50 | 16 |
| 20.6 | 4.32 | 92 |
| 21.1* | 4.20 | 13 |
| 22.4 | 3.97 | 22 |
| 22.6* | 3.94 | 5 |
| 23.1 | 3.85 | 6 |
| 25.2 | 3.534 | 28 |
| 25.8** | 3.454 | 32 |
| 27.6 | 3.237 | 4 |
| 28.4 | 3.142 | 4 |
| 29.0* | 3.079 | 5 |
| 29.5 | 3.025 | 4 |
| 29.9* | 2.987 | 7 |
| 30.5** | 2.930 | 37 |
| 31.3 | 2.863 | 25 |
| 32.4 | 2.767 | 26 |
| 34.4** | 2.608 | 11 |
| 35.4* | 2.537 | 5 |
| 36.3 | 2.473 | 5 |

| 2θ | d, (Å) | 100xI/Io |
|---|---|---|
| 37.8* | 2.382 | 4 |
| 38.7* | 2.329 | 6 |
| 38.8 | 2.323 | 6 |
| 39.6 | 2.276 | 5 |
| 43.3 | 2.088 | 5 |
| 45.1* | 2.010 | 3 |
| 46.1* | 1.971 | 4 |
| 46.3* | 1.962 | 5 |
| 47.2 | 1.924 | 7 |
| 48.7* | 1.870 | 6 |
| 48.9 | 1.863 | 6 |
| 51.0 | 1.791 | 4 |
| 53.0 | 1.728 | 4 |
| 53.1 | 1.726 | 4 |

* peak may contain impurity
** peak contains impurity and CoMnMgAPSO-34

(b) A portion of the as-synthesized CoMnMgAPSO—34 of part (a) was calcined in air at 600°C for one (1) hour. The calcined product was characterized by the x-ray powder diffraction pattern below:

| 2θ | d, (Å) | 100xI/Io |
|---|---|---|
| 7.5* | 11.76 | 100 |
| 9.7 | 9.14 | 86 |
| 13.1** | 6.79 | 18 |
| 15.0* | 5.90 | 30 |
| 16.3 | 5.44 | 8 |
| 18.1 | 4.90 | 7 |
| 19.9* | 4.47 | 19 |
| 21.2** | 4.19 | 35 |
| 21.5 | 4.13 | 44 |
| 22.6* | 3.94 | 37 |
| 23.0 | 3.87 | 6 |
| 26.1** | 3.414 | 21 |
| 26.4 | 3.379 | 9 |
| 29.2* | 3.060 | 8 |
| 30.2* | 2.956 | 59 |
| 31.2 | 2.871 | 12 |
| 31.7 | 2.819 | 7 |
| 34.7* | 2.582 | 13 |
| 35.5 | 2.528 | 16 |

* peak may contain impurity
** peak contains impurity and CoMnMgAPSO-34

(c) The species CoMnMgAPSO—34 is a molecular sieve having three-dimensional microporous framework structure of $CoO_2^{-2}$, $MnO_2^{-2}$, $MgO_2^{-2}$, $AlO_2^-$, $PO_2^+$, and $SiO_2$ tetrahedral oxide units and have an empirical chemical composition on an anhydrous basis expressed by the formula:

$$mR:(Co_tMn_uMg_vAl_xP_ySi_z)O_2$$

wherein "R" represents an organic templating agent present in the intracrystalline pore system; "m" represents the molar amount of "R" present per mole of $(Co_tMn_uMg_vAl_xP_ySi_z)O_2$ and has a value of from zero to about 0.3; and "t", "u", "v", "x", "y", and "z", where "w" is the sum of "t + u + v", represent the mole fractions of cobalt, manganese, magnesium, aluminum, phosphorus and silicon, respectively, present as tetrahedral oxides, said mole fractions being within the compositional area defined by points A, B, C, D and E of Fig. 1, more preferably by the compositional area defined by points a, b, c, d, e and f of Fig. 2, and having a characteristic x-ray powder diffraction pattern which contains at least the d-spacings set forth below:

| 2θ | d, (Å) | Relative Intensity |
|---|---|---|
| 9.3-9.8 | 9.51-9.03 | m-vs |
| 12.6-13.2 | 7.03-6.71 | w-m |
| 15.8-16.3 | 5.61-5.44 | vw-m |
| 20.25-21.2 | 4.39-4.19 | w-vs |
| 24.8-25.4 | 3.59-3.507 | vw-m |
| 30.0-30.9 | 2.979-2.894 | vw-m |

(d) The CoMnMgAPSO—5 compositions for which x-ray powder diffraction data have been obtained have patterns which are characterized by the data set forth below:

| 2θ | d, (Å) | 100xI/Io |
|---|---|---|
| 9.5-9.7 | 9.31-9.14 | 100 |
| 12.8-13.1 | 6.90-6.79 | 13-19 |
| 14.1 | 6.28 | 12-14 |
| 16.0-16.3 | 5.54-5.44 | 31-46 |
| 18.0-18.1 | 4.93-4.90 | 21-28 |
| 19.2 | 4.62 | 5-12 |
| 20.5-21.2 | 4.33-4.19 | 61-92 |
| 21.5 | 4.13 | 44 |
| 22.4 | 3.97 | 4-25 |
| 23.0-23.1 | 3.87-3.85 | 4-6 |
| 25.2 | 3.534 | 21-28 |
| 25.8-26.1 | 3.453-3.414 | 13-32 |
| 26.4 | 3.379 | 9 |
| 27.6 | 3.237 | 4 |
| 28.4 | 3.142 | 4-5 |
| 29.5 | 3.025 | 4 |
| 30.2-30.5 | 2.960-2.930 | 21-37 |
| 31.2-31.3 | 2.871-2.863 | 14-25 |
| 31.7 | 2.819 | 7 |
| 32.4 | 2.767 | 15-26 |
| 34.4 | 2.608 | 5-11 |

| 2θ | d, (Å) | 100xI/Io |
|---|---|---|
| 35.5 | 2.528 | 16 |
| 36.3 | 2.473 | 4-5 |
| 38.8 | 2.323 | 6 |
| 39.6 | 2.276 | 5 |
| 43.3 | 2.088 | 5 |
| 47.2-47.5 | 1.924-1.916 | 4-7 |
| 48.9 | 1.863 | 4-6 |
| 51.0 | 1.791 | 4 |
| 53.0 | 1.728 | 4 |
| 53.1 | 1.726 | 4 |

Example 21
(Preparation of MgBeMnAPSO—5)

a) MgBeMnAPSO—5 may be prepared from a reaction mixture having a composition, expressed in terms of the molar oxide ratios of the components of the reaction mixture, of:

$$1.0—2.0\ TPA:0.1—0.4\ MgO:0.1—0.4\ BeO:0.1—0.4\ MnO:0.5—1.0\ Al_2O_3:0.5—1.0\ P_2O_5:\ 0.1—0.6\ SiO_2:40—100\ H_2O$$

where "TPA' denotes tripropylamine.

The reaction mixture is digested by placing the reaction mixture in a sealed stainless steel pressure vessel and heating it at an effective temperature and for an effective time to produce MgBeMnAPSO—5 product. Solids are recovered by filtration, washed with water and dried in air at room temperature.

The MgBeMnAPSO—5 product's chemical analysis shown the product contains magnesium, beryllium, manganese, aluminum, phosphorus and silicon in amounts within the pentagonal compositional area defined by points A, B, C, D and E of Fig. 1.

The x-ray powder diffraction pattern of a MgBeMnAPSO—5 product is characterized by the following data:

| 2θ | d (Å) | Relative Intensity |
|---|---|---|
| 7.2-7.7 | 12.28-11.48 | m-vs |
| 19.4-19.9 | 4.58-4.46 | w-m |
| 20.85-21.3 | 4.26-4.17 | w-vs |
| 22.1-22.6 | 4.02-3.93 | m-vs |
| 25.6-26.1 | 3.480-3.414 | vw-m |

b) the x-ray powder diffraction pattern for a calcined MgBeMnAPSO—5 is also characterized by the x-ray pattern of part a).

c) When the calcined MgBeMnAPSO—5 of part (b) is utilized in adsorption capacity studies using a standard McBain-Baker gravimetric adsorption apparatus the measurements are made on a sample after activation at 350°C in vacuum. The following data are used in the adsorption studies:

| Adsorbate | Kinetic Diameter (Å) | Pressure (Torr)(x1.333 mbar) | Temp, °C | Wt. % Adsorbed* |
|---|---|---|---|---|
| $O_2$ | 3.46 | 100 | -183 | 7 |
| $O_2$ | 3.46 | 750 | -183 | 10 |
| Neopentane | 6.2 | 700 | 24 | 4 |
| $H_2O$ | 2.65 | 4.3 | 24 | 4 |
| $H_2O$ | 2.65 | 20.0 | 24 | 12 |

*typical amount adsorbed

The pore diameter of MgBeMnAPSO—5 is greater than 6.2Å.

Example 22

(Preparation of FeGaMgAPSO—11)

a) FeGaMgAPSO—11 may be prepared from a reaction mixture having a composition, expressed in terms of the molar oxide ratios of the components of the reaction mixture, of:

1.0—2.0 DPA:0.1—0.4 FeO:0.1—0.4 MgO:0.5—0.2 $Ga_2O_3$:0.5—1.0 $Al_2O_3$:
0.5—1.0 $P_2O_5$:0.1—0.6 $SiO_2$:40—100 $H_2O$

where "DPA" denotes di-n-propylamine.

The reaction mixture is digested by placing the reaction mixture in a sealed stainless steel pressure vessel and heating it at an effective temperature and for an effective to produce FeGaMgAPSO—11 product. Solids are then recovered by filtration, washed with water and dried in air at room temperature.

The FeGaMgAPSO—11 product's chemical analysis shows the FeGaMgAPSO—11 product contains iron, gallium, magnesium, aluminum, phosphorus and silicon in amounts within the pentagonal compositional area defined by points A, B, C, D and E of Fig. 1.

The x-ray powder diffraction pattern of a FeGaMgAPSO—11 product is characterized by the following data:

| 2θ | d (Å) | Relative Intensity |
|---|---|---|
| 7.8- 8.2 | 11.19-10.85 | m-s |
| 9.0- 9.8 | 9.83-9.03 | vw-vs |
| 12.8-13.6 | 6.92-6.51 | vw-m |
| 19.9-20.5 | 4.46-4.33 | m-s |
| 20.8-21.8 | 4.27-4.08 | m-vs |
| 22.0-22.6 | 4.04-3.93 | m-vs |
| 22.6-23.1 | 3.93-3.85 | vw-vs |
| 23.1-23.5 | 3.85-3.79 | w-vs |

b) The x-ray powder diffraction pattern for a calcined FeGaMgAPSO—11 is also characterized by the x-ray pattern of part a).

c) When the calcined FeGaMgAPSO—11 of part (b) is utilized in adsorption capacity studies using a standard McBain-Baker gravimetric adsorption apparatus the measurements are made on a sample after activation at 350°C in vacuum. The following data are used in the adsorption studies:

| Adsorbate | Kinetic Diameter (Å) | Pressure (Torr)(x 1.333 mbar) | Temp, °C | Wt. % Adsorbed* |
|---|---|---|---|---|
| $O_2$ | 3.46 | 100 | -183 | 5 |
| $O_2$ | 3.46 | 750 | -183 | 6 |
| Cyclohexane | 6.0 | 90 | 24 | 4 |
| $H_2O$ | 2.65 | 4.3 | 24 | 6 |
| $H_2O$ | 2.65 | 20 | 24 | 8 |

*typical amount adsorbed

The pore diameter of FeGaMgAPSO—11 is about 6Å.

Example 23

(Preparation of ZnCoMnAPSO—34)

a) ZnCoMnAPSO—34 may be prepared from a reaction mixture having a composition, expressed in terms of the molar oxide ratios of the components of the reaction mixture, of:

1.0—2.0 TEAOH:0.1—0.4 ZnO:0.1—0.4 CoO:0.1—0.4 MnO:0.5—1.0 $Al_2O_3$:
0.5—1.0 $P_2O_5$:0.1—0.6 $SiO_2$:40—100 $H_2O$

where "TEAOH" denotes tetraethylammonium hydroxide.

The reaction mixture is digested by placing the reaction mixture in a sealed stainless steel pressure vessel and heating it at an effective temperature and for an effective time to produce ZnCoMnAPSO—34 product. The solids are recovered by filtration, washed with water and dried in air at room temperature.

The ZnCoMnAPSO—34 product's chemical analysis shows the ZnCoMnAPSO—34 product contains zinc, cobalt, manganese, aluminum, phosphorus and silicon in amounts within the pentagonal compositional area defined by points A, B, C, D and E of Fig. 1.

The x-ray powder diffraction pattern of a ZnCoMnAPSO—34 product is characterized by the following data:

| 2θ | d (Å) | Relative Intensity |
|---|---|---|
| 9.3-9.8 | 9.51-9.03 | m-vs |
| 12.6-13.2 | 7.03-6.71 | w-m |
| 15.8-16.3 | 5.61-5.44 | vw-m |
| 20.25-21.2 | 4.39-4.19 | w-vs |
| 24.8-25.4 | 3.59-3.507 | vw-m |
| 30.0-30.9 | 2.979-2.894 | vw-m |

b) The x-ray powder diffraction pattern for a calcined ZnCoMnAPSO—34 is characterized by the x-ray pattern of part a).

c) When the calcined ZnCoMnAPSO—34 of part (b) is utilized in adsorption capacity studies using a standard McBain-Baker gravimetric adsorption apparatus the measurements are made on a sample after activation at 350°C in vacuum. The following data are used in the adsorption studies:

| Adsorbate | Kinetic Diameter (Å) | Pressure (Torr)(x 1.333 mbar) | Temp, °C | Wt. % Adsorbed* |
|---|---|---|---|---|
| $O_2$ | 3.46 | 100 | -183 | 13 |
| $O_2$ | 3.46 | 750 | -183 | 18 |
| n-hexane | 4.3 | 100 | 24 | 6 |
| $H_2O$ | 2.65 | 4.3 | 24 | 15 |
| $H_2O$ | 2.65 | 20 | 24 | 21 |

*typical amount adsorbed

The pore diameter of ZnCoMnAPSO-34 is about 4.3.Å

Example 24

The catalytic activity of the CoMnMgAPSO compositions of examples 13 and 14 were evaluated in n-butane cracking using a bench-scale apparatus.

The reactor was a cylindrical quartz tube 254 mm. in length and 10.3 mm. I.D. In each test the reactor was loaded with particles of the test CoMnMgAPSO's which were 20—40 mesh (U.S. Std.) in size and in an amount of from 0.5 to 5 grams, the quantity being selected so that the conversion of n-butane was at least 5% and not more than 90% under the test conditions. The CoMnMgAPSO samples were calcined in air at 600°C for 1.5 hours to remove organic materials from the pore system, and were activated *in situ* in the reactor in a flowing stream of helium at 500°C for one hour. The feedstock was a helium-n-butane mixture containing 2 mole percent n-butene and was passed through the reactor at a rate of 50 cc./minute. Analysis of the feedstock and the reactor effluent were carried out using conventional gas chromatography techniques. The reactor effluent was analyzed after 10 minutes of on-stream operation. From the analytical data the pseudo-first-order rate constants ($k_A$) were calculated and are set forth below:

| Product of Ex. No: | Rate Contant ($k_A$)** |
|---|---|
| 13* | 8.8 |
| 14* | 0.2 |

* calcined at 600°C in air for 1.5 hours

** ($cm^3$/gram minute)

Process Applications for Senapsos

The compositions of the present invention are, in general, hydrophilic and adsorb water preferentially over common hydrocarbon molecules such as paraffins, olefins and aromatic species, e.g., benzene, xylenes and cumene. Thus the present compositions as a class are useful as desiccants in such adsorption separation/purification processes as natural gas drying, cracked gas drying. Water is also preferentially adsorbed over the so-called permanent gases such as carbon dioxide, nitrogen, oxygen and hydrogen. These are therefore suitably employed in the drying of reformer hydrogen streams and in the drying of oxygen, nitrogen or air prior to liquidication.

The present compositions also exhibit novel surface selectivity characteristics which render them useful as catalyst or catalyst bases in a number of hydrocarbon conversion and oxidative combustion reactions. They can be impregnated or otherwise loaded with catalytically active metals by methods well known in the art and used, for example, in fabricating catalyst compositions having silica or alumina bases. Of the general class, those species having pores larger than about 4Å are preferred for catalytic applications.

Among the hydrocarbon conversion reactions catalyzed by compositions are cracking, hydrocracking, alkylation for both the aromatic and isoparaffin types, isomerization including xylene isomerization, polymerization, reforming, hydrogenation, dehydrogenation, transalkylation, dealkylation, hydrodecyclization and dehydrocyclization.

Using catalyst compositions which contain a hydrogenation promoter such as platinum or palladium, heavy petroleum residual stocks, cyclic stocks and other hydrocrackable charge stocks, can be hydrocracked at temperatures in the range of 400°F to 825°F (204.4—440.6°C) using molar ratios of hydrogen to hydrocarbon in the range of between 2 and 80, pressures between 10 and 3500 p.s.i.g. (1.7—242.2 bar), and a liquid hourly space velocity (LHSV) of from 0.1 to 20, preferably 1.0 to 10.

The catalyst compositions employed in hydrocracking are also suitable for use in reforming processes in which the hydrocarbon feedstocks contact the catalyst at temperatures of from about 700°F to 1000°F (371—538°C), hydrogen pressures of from 100 to 500 p.s.i.g. (7.9—35.5 bar), LHSV values in the range of 0.1 to 10 and hydrogen to hydrocarbon molar ratios in the range of 1 to 20, preferably between 4 and 12.

These same catalysts, i.e. those containing hydrogenation promoters, are also useful in hydroisomerizations processes in which feedstocks such as normal paraffins are converted to saturated branched chain isomers. Hydroisomerization is carried out at a temperature of from about 200°F to 600°F (93.3—315.5°C), preferably 300°F to 550°F (149—288°C) with an LHSV value of from about 0.2 to 1.0. Hydrogen is supplied to the reactor in admixture with the hydrocarbon feedstock in molar proportions (hydrogen/hydrocarbon) of between 1 and 5.

At somewhat higher temperatures, i.e. from about 650°F to 1000°F (454—510°C), preferably 850°F to 950°F (2—4.5 bar) and usually at somewhat lower pressures within the range of about 15 to 50 p.s.i.g., the same catalyst compositions are used to hydroisomerize normal paraffins. Preferably the paraffin feedstock comprises normal paraffins having a carbon number range of $C_7$—$C_{20}$. Contact time between the feedstock and the catalyst is generally relatively short to avoid undesirable side reactions such as olefin polymerization and paraffin cracking. LHSV values in the range of 0.1 to 10, preferably 1.0 to 6.0 are suitable.

The unique crystal structure of the present catalysts and their availability in a form totally void of alkali metal content favor their use in the conversion of alkylaromatic compounds, particularly the catalytic disproportionation of toluene, ethylene, trimethyl benzenes, tetramethyl benzenes and the like. In the disproportionation process, isomerization and transalkylation can also occur.

Group VIII noble metal adjuvants alone or in conjunction with Group VI—B metals such as tungsten, molybdenum and chromium are preferably included in the catalyst composition in amounts of from about 3 to 15 weight-% of the overall composition. Extraneous hydrogen can, but need not, be present in the reaction zone which is maintained at a temperature of from about 400 to 750°F (204—399°C), pressures in the range of 100 to 2000 p.s.i.g. (7.9—138.8 bar) and LHSV values in the range of 0.1 to 15.

Catalytic cracking processes are preferably carried out with compositions using feedstocks such as gas oils, heavy naphthas, deasphalted crude oil residua, etc., with gasoline being the principal desired product. Temperature conditions of 850 to 1100°F (454—593°C), LHSV values of 0.5 to 10 and pressure conditions of from about 0 to 50 p.s.i.g. (1.0—4.5 bar) are suitable.

Dehydrocyclization reactions employing paraffinic hydrocarbon feedstocks, preferably normal paraffins having more than 6 carbon atoms, to form benzene, xylenes, toluene and the like are carried out using essentially the same reaction conditions as for catalytic cracking. For these reactions it is preferred to use the catalyst in conjunction with a Group VIII non-noble metal cation such as cobalt and nickel.

In catalytic dealkylation wherein it is desired to cleave paraffinic side chains from aromatic nuclei without substantially hydrogenating the ring structure, relatively high temperatures in the range of about 800°—1000°F (427—538°C) are employed at moderate hydrogen pressures of about 300—1000 p.s.i.g. (21.7—69.9 bar), other conditions being similar to those described above for catalytic hydrocracking. Preferred catalysts are of the same type described above in connection with catalytic dehydrocyclization. Particularly desirable dealkylation reactions contemplated herein include the conversion of methylnaphthalene to naphthalene and toluene and/or xylenes to benzene.

In catalytic hydrofining, the primary objective is to promote the selective hydrodecomposition of

organic sulfur and/or nitrogen compounds in the feed, without substantially affecting hydrocarbon molecules therein. For this purpose it is preferred to employ the same general conditions described above for catalytic hydrocracking, and catalysts of the same general nature described in connection with dehydrocyclization operations. Feedstocks include gasoline fractions, kerosenes, jet fuel fractions, diesel fractions, light and heavy gas oils, deasphalted crude oil residue and the like any of which may contain up to about 54 weight-percent of sulfur and up to about 3 weight-percent of nitrogen.

Similar conditions can be employed to effect hydrofining, i.e., denitrogenation and desulfurization, of hydrocarbon feeds containing substantial proportions of organonitrogen and organosulfur compounds. It is generally recognized that the presence of substantial amounts of such constituents markedly inhibits the activity of hdyrocracking catalysts. Consequently, it is necessary to operate at more extreme conditions when it is desired to obtain the same degree of hydrocracking conversion per pass on a relatively nitrogenous feed than are required with a feed containing less organonitrogen compounds. Consequently, the conditions under which denitrogenation, desulfurization and/or hydrocracking can be most expeditiously accomplished in any given situation are necessarily determined in view of the characteristics of the feedstocks in particular the concentration of organonitrogen compounds in the feedstock. As a result of the effect of organonitrogen compounds on the hydrocracking activity of these compositions it is not at all unlikely that the conditions most suitable for denitrogenation of a given feedstock having a relatively high organonitrogen content with minimal hydrocracking, e.g., less than 20 volume percent of fresh feed per pass, might be the same as those preferred for hydrocracking another feedstock having a lower concentration of hydrocracking inhibiting constituents e.g., organonitrogen compounds. Consequently, it has become the practice in this art to establish the conditions under which a certain feed is to be contacted on the basis of preliminary screening tests with the specific catalyst and feedstock.

Isomerization reactions are carried out under conditions similar to those described above for reforming, using somewhat more acidic catalysts. Olefins are preferably isomerized at temperatures of 500°—900°F (260—482°C), while paraffins, naphthenes and alkyl aromatics are isomerized at temperatures of 700°—1000°F. Particularly desirable isomerization reactions contemplated herein include the conversion of n-heptene and/or n-octane to isoheptanes, iso-octanes, butane to iso-butane, methylcyclopentane to cyclohexane, meta-xylene and/or ortho-xylene to paraxylene, 1-butene to 2-butene and/or isobutene, n-hexane to isohexene, cyclohexane to methylcyclopentene etc. The preferred form of the catlayst is a combination of the with polyvalent metal compounds (such as sulfides) of metals of Group II—A, Group II—B and rare earth metals. For alkylation and dealkylation processes and SENAPSO compositions having pores of at least 5Å are preferred. When employed for dealkylation of alkyl aromatics, the temperature is usually at least 350°F (176.7°C) and ranges up to a temperature at which substantial cracking of the feedstock or conversion products occurs, generally up to about 700°F (371.1°C). The temperature is preferably at least 450°F (232.2°C) and not greater than the critical temperature of the compound undergoing dealkylation. Pressure conditions are applied to retain at least the aromatic feed in the liquid state. For alkylation the temperature can be as low as 250°F (121.1°C) but is preferably at least 350°F (176.7°C). In the alkylation of benzene, toluene and xylene, the preferred alkylating agents are olefins such as ethylene and propylene.

## Claims

1. Crystalline molecular sieves having three-dimensional microporous framework structures of $MO_2$, $AlO_2^-$ and $PO_2^+$ and $SiO_2$ tetrahedral oxide units and having an empirical chemical composition on an anhydrous basis expressed by the formula:

$$mR: (M_wAl_xP_ySi_z)O_2$$

wherein R'' represents at least one organic templating agent present in the intracrystalline pore system; "m" represents he molar amount of "R" present per mole of $(M_wAl_xP_ySi_z)O_2$ and has a value of from zero (0) to about 0.3; "M" represents at least two elements selected from the group consisting of arsenic, beryllium, boron, chromium, cobalt, gallium, germanium, iron, lithium, magnesium, manganese, titanium, vanadium and zinc; "n" may be −3, −2, −1,0 or +1 depending on the oxidation state of "M" and "w", "x", "y" and "z" (where "w" is the sum of the individual mole fractions "$W_1$", "$W_2$" and "$W_3$" etc. and each element has a mole fraction of at least 0.01) represent the mole fractions of elements "M", aluminium, phosphorus and silicon, respectively, present as tetrahedral oxides, being such that they are within the pentagonal compositional area defined by points A, B, C, D and E of Fig. 1, "w", "x", "y" and "z" having the following values:

| | Mole Fraction | | |
|---|---|---|---|
| Point | x | y | (z+w) |
| A | 0.60 | 0.37 | 0.03 |
| B | 0.37 | 0.60 | 0.03 |
| C | 0.01 | 0.60 | 0.39 |
| D | 0.01 | 0.01 | 0.98 |
| E | 0.60 | 0.01 | 0.39 |

2. Crystalline molecular sieves according to claim 1 wherein "w", "x", "y" and "z" are within the tetragonal compositional area defined by points a, b, c, d, e and f of Fig. 2 and "w", "y", "y" and "z" have the following values:

| | Mole Fraction | | |
|---|---|---|---|
| Point | x | y | (w+z) |
| a | 0.60 | 0.37 | 0.03 |
| b | 0.37 | 0.60 | 0.03 |
| c | 0.01 | 0.60 | 0.39 |
| d | 0.01 | 0.39 | 0.60 |
| e | 0.39 | 0.01 | 0.60 |
| f | 0.60 | 0.01 | 0.39 |

3. The crystalline molecular sieves of claims 1 or 2 having a characteristic x-ray powder diffraction pattern which contains at least the d-spacings set forth in the following table:

| 2θ | d (Å) | Relative Intensity |
|---|---|---|
| 7.2 - 7.7 | 12.28 - 11.48 | m-vs |
| 19.4 - 19.9 | 4.58 - 4.46 | w-m |
| 20.85 - 21.3 | 4.26 - 4.17 | w-vs |
| 22.1 - 22.6 | 4.02 - 3.93 | m-vs |
| 25.6 - 26.1 | 3.480 - 3.414 | vw-m |

4. The crystalline molecular sieves of claims 1 or 2 having a characteristic x-ray powder diffraction pattern which contains at least the following d-spacings:

| 2θ | d (Å) | Relative Intensity |
|---|---|---|
| 7.8 - 8.2 | 11.19 - 10.85 | m-s |
| 9.0 - 9.8 | 9.83 - 9.03 | vw-vs |
| 12.8 - 13.6 | 6.92 - 6.51 | vw-m |
| 19.9 - 20.5 | 4.46 - 4.33 | m-s |
| 20.8 - 21.8 | 4.27 - 4.08 | m-vs |
| 22.0 - 22.6 | 4.04 - 3.93 | m-vs |
| 22.6 - 23.1 | 3.93 - 3.85 | vw-vs |
| 23.1 - 23.5 | 3.85 - 3.79 | w-vs |

5. The crystalline molecular sieves of claims 1 or 2 having a characteristic x-ray powder diffraction pattern which contains at least the following d-spacings:

| 2θ | d (Å) | Relative Intensity |
|---|---|---|
| 11.3 - 11.6 | 7.83 - 7.63 | w-vs |
| 18.55 - 18.9 | 4.78 - 4.70 | vm-m |
| 21.85 - 22.2 | 4.07 - 4.00 | m-vs |
| 22.8 - 23.3 | 3.900 - 3.818 | w-m |
| 26.4 - 27.3 | 3.370 - 3.267 | w-m |
| 29.6 - 29.9 | 3.018 - 2.988 | w-m |

6. The crystalline molecular sieves of claims 1 or 2 having a characteristic x-ray powder diffraction pattern which contains at least the following d-spacings:

| 2θ | d (Å) | Relative Intensity |
|---|---|---|
| 13.8 - 14.2 | 6.42 - 6.23 | m-vs |
| 19.6 - 20.15 | 6.53 - 4.41 | m |
| 24.1 - 24.7 | 3.695 - 3.603 | m-vs |
| 27.9 - 28.6 | 3.198 - 3.121 | w |
| 31.3 - 32.05 | 2.861 - 2.791 | w |
| 34.35 - 35.0 | 2.610 - 2.601 | w-m |

7. The crystalline molecular sieves of claims 1 or 2 having a characteristic x-ray powder diffraction pattern which contains at least the following d-spacings:

| 2θ | d (Å) | Relative Intensity |
|---|---|---|
| 8.4 - 9.5 | 10.53 - 9.31 | w-s |
| 20.2 - 20.4 | 4.40 - 4.35 | m |
| 22.0 - 22.1 | 4.040 - 4.022 | m |
| 22.5 - 22.7 | 3.952 - 3.92 | vs |
| 31.6 - 31.8 | 2.831 - 2.814 | w-m |

8. The crystalline molecular sieves of claims 1 or 2 having a characteristic x-ray powder diffraction pattern which contains at least the following d-spacings:

| 2θ | d (Å) | Relative Intensity |
|---|---|---|
| 9.3 - 9.8 | 9.51 - 9.03 | m-vs |
| 12.6 - 13.2 | 7.03 - 6.71 | w-m |
| 15.8 - 16.3 | 5.61 - 5.44 | vw-m |
| 20.25 - 21.2 | 4.39 - 4.19 | w-vs |
| 24.8 - 25.4 | 3.59 - 3.507 | vw-m |
| 30.0 - 30.9 | 2.979 - 2.894 | vw-m |

9. The crystalline molecular sieves of claims 1 or 2 having a characteristic x-ray powder diffraction pattern which contains at least the following d-spacings:

| 2θ | d (Å) | Relative Intensity |
|---|---|---|
| 10.6 - 11.1 | 8.35 - 7.97 | vw-vs |
| 13.1 - 13.7 | 6.76 - 6.46 | vw-vs |
| 17.0 - 17.6 | 5.22 - 5.04 | w-s |
| 20.6 - 21.25 | 4.31 - 4.18 | vw-m |
| 21.6 - 22.3 | 4.11 - 3.99 | m-vs |
| 28.1 - 28.8 | 3.175 - 3.100 | vw-m |

10. The crystalline molecular sieves of claims 1 or 2 having a characteristic x-ray powder diffraction pattern which contains at least the following d-spacings:

| 2θ | d (Å) | Relative Intensity |
|---|---|---|
| 7.45 - 8.0 | 11.14 - 11.05 | vs |
| 8.1 - 8.3 | 10.91 - 10.65 | w-m |
| 16.3 - 16.6 | 5.44 - 5.34 | w-m |
| 18.9 - 19.4 | 4.70 - 4.57 | w-m |
| 20.7 - 21.0 | 4.29 - 4.23 | w-m |

11. The crystalline molecular sieves of claims 1 or 2 having a characteristic x-ray powder diffraction pattern which contains at least the following d-spacings:

| 2θ | d (Å) | Relative Intensity |
|---|---|---|
| 9.2 - 9.6 | 9.61 - 9.21 | m |
| 13.1 - 13.5 | 6.76 - 6.56 | m |
| 17.8 - 18.4 | 4.98 - 4.82 | w-m |
| 20.8 - 21.3 | 4.27 - 4.17 | m-vs |
| 22.2 - 22.85 | 4.00 - 3.892 | m-vs |
| 26.4 - 27.05 | 3.376 - 3.296 | w-m |

12. The crystalline molecular sieves of claims 1 or 2 having a characteristic x-ray powder diffraction pattern which contains at least the following d-spacings:

| 2θ | d (Å) | Relative Intensity |
|---|---|---|
| 12.3 - 12.95 | 7.20 - 6.83 | m-vs |
| 16.8 - 17.45 | 5.28 - 5.09 | vw-w |
| 21.45 - 21.85 | 4.145 - 4.071 | m-vs |
| 27.1 - 27.85 | 3.291 - 3.232 | w-vs |
| 32.4 - 33.2 | 2.763 - 2.699 | vw-m |

13. The crystalline molecular sieves of claims 1 or 2 having a characteristic x-ray powder diffraction pattern which contains at least the following d-spacings:

| 2θ | d (Å) | Relative Intensity |
|---|---|---|
| 9.2 - 9.6 | 9.61 - 9.21 | m-vs |
| 15.9 - 16.3 | 5.57 - 5.44 | vw-m |
| 20.5 - 21.0 | 4.33 - 4.23 | m-vs |
| 24.3 - 25.1 | 3.66 - 3.548 | w-m |
| 30.5 - 31.1 | 2.931 - 2.876 | vw-m |

14. The crystalline molecular sieves of claims 1 or 2 having a characteristic x-ray powder diffraction pattern which contains at least the following d-spacings:

| 2θ | d (Å) | Relative Intensity |
|---|---|---|
| 7.2 - 8.1 | 12.28 - 10.92 | vs |
| 12.9 - 13.6 | 6.86 - 6.51 | vw |
| 21.2 - 22.2 | 4.19 - 4.501 | vw-m |
| 22.5 - 23.45 | 3.95 - 3.793 | vw-m |
| 26.6 - 27.9 | 3.351 - 3.198 | vw-m |

15. The crystalline molecular sieves of claims 1 or 2 having a characteristic x-ray powder diffraction pattern which contains at least the following d-spacings:

| 2θ | d (Å) | Relative Intensity |
|---|---|---|
| 9.4 - 9.6 | 9.41 - 9.21 | vs |
| 12.8 - 13.1 | 6.92 - 6.76 | vw-m |
| 16.0 - 16.3 | 5.54 - 5.44 | vw-m |
| 20.5 - 21.0 | 4.31 - 4.23 | m-vs |
| 24.6 - 25.3 | 3.613 - 3.526 | vm-m |
| 30.6 - 31.1 | 2.921 - 2.876 | vw-m |

16. The crystalline molecular sieves of claims 1 or 2 wherein "M" is selected from the group consisting of magnesium, manganese, titanium, vanadium, zinc, cobalt, iron and beryllium.

17. The crystalline molecular sieves of claims 1 or 2 wherein two elements "M" are present.

18. The crystalline molecular sieves of claim 17 wherein "M" is cobalt and manganese.

19. The crystalline molecular sieves of claim 1 or 2 wherein three elements "M" are present.

20. The crystalline molecular sieves of claim 19 wherein "M" is cobalt, manganese and magnesium.

21. Process for preparing the crystalline molecular sieves of claim 1 having three-dimensional framework structures wherein said process comprises heating at an effective temperature between 50°C and 250°C a reaction mixture composition expressed in terms of molar oxide ratios as follows:

$$aR: (M_wAl_xP_ySi_z)O_2 : bH_2O$$

wherein "R" is an organic templating agent; "a" is the amount of "R" and may be zero or an effective amount greater than zero to about 6; "b" has a value of from zero to about 500; "M" represents at least two elements selected from the group consisting of arsenic, beryllium, boron, chromium, cobalt, gallium, germanium, iron, lithium, magnesium, manganese, titanium, vanadium and zinc; and "w", "x", "y" and "z" represent the mole fractions, respectively, of elements "M", aluminum and phosphorous and silicon in

the $(M_wAl_xP_ySi_z)O_2$ constituent, and the mole fraction of each element, aluminum, phosphorus and silicon has a value of at least 0.01, for a period of time which is effective to produce crystals of the molecular sieves of claim 1.

22. Process according to claim 21 where "w", "x", "y" and "z" are within the area defined by points F, G, H, I and J of Fig. 3 and "w", "x", "y" and "z" have the following values:

| | Mole Fraction | | |
|---|---|---|---|
| Point | x | y | (z+w) |
| F | 0.60 | 0.37 | 0.03 |
| G | 0.37 | 0.60 | 0.03 |
| H | 0.01 | 0.60 | 0.39 |
| I | 0.01 | 0.01 | 0.98 |
| J | 0.60 | 0.01 | 0.39 |

23. Process according to claim 21 wherein the source of phosphorus in the reaction mixture is orthophosphoric acid.

24. Process according to claim 21 wherein the source of phosphorus in the reaction mixture is orthophosphoric acid and the source of aluminum is at least one compound selected from the group of pseudo-boehmite and aluminum alkoxide.

25. Process according to claim 24 wherein the aluminum alkoxide is aluminum isoproproxide.

26. Process according to claim 21 wherein the source of silicon is silica.

27. Process according to claim 21 wherein the reactive sources of elements "M" are selected from the group consisting of oxides, hydroxides, alkoxides, nitrates, sulfates, borates, germanates, vanadates, halides, carboxylates and mixtures thereof.

28. Process according to claim 21 or claim 22 wherein the organic templating agent is a quaternary ammonium or quaternary phosphonium compound having the formula:

$$R_4X^+$$

wherein X is nitrogen or phosphorus and each R is an alkyl or aryl group containing from 1 to 8 carbon atoms.

29. Process according to claim 21 wherein the organic templating agent is an amine.

30. Process according to claim 21 or claim 22 wherein the templating agent is selected from the group consisting of tetrapropylammonium ion; tetraethylammonium ion tripropylamine; triethylamine; triethanolamine; piperidine; cyclohexylamine; 2-methyl pyridine; N,N-dimethylbenzylamine; N,N-dimethylethanolamine; chlorine, N,N-dimethylpiperazine; 1,4-diazabicyclo-(2,2,2) octane; N-methyldiethanolamine; N-methylethanolamine; N-methylpiperidine; 3-methylpiperidine; N-methylcyclohexylamine; 3-methylpyridine; 4-methylpyridine; quinuclidine; N,N'-dimethyl-1,4-diazabicyclo (2,2,2) octane ion; tetramethylammonium ion; tetrabutylammonium ion; tetrapentylammonium ion; di-n-butylamine; neopentylamine; di-n-pentylamine; isopropylamine; t-butylamine; ethylenediamine; pyrrolidine; 2-imidazolidone; di-n-propylamine; and a polymeric quaternary ammonium salt $[(C_{14}H_{32}N_2)(OH)_2]_x$ wherein x is a value of at least 2.

31. Molecular sieves prepared by calcining the compositions of claim 1 or claim 2 at a temperature sufficiently high to remove at least some of any organic templating agent present in the intracrystalline pore system.

32. Process for separating molecular species from admixture with molecular species having a lesser degree of polarity which comprises contacting said mixture of molecular species with a molecular sieve of claim 1 or claim 2 having pore diameters large enough to adsorb at least one of the more polar molecular species, said molecular sieve being at least partially activated whereby molecules of the more polar molecular species are selectively adsorbed into the intracrystalline pore system thereof.

33. Process for separating a mixture of molecular species having different kinetic diameters which comprises contacting said mixture with a molecular sieve of claim 1 or claim 2 having pore diameters large enough to adsorb at least one but not all molecular species of said mixture, said molecular sieve being at least partially activated whereby at least some molecules whose kinetic diameters are sufficiently small can enter the intracrystalline pore system thereof.

34. Process according to claim 32 wherein the more polar molecular species is water.

35. Process for converting a hydrocarbon which comprises contacting said hydrocarbon under hydrocarbon converting conditions with a molecular sieve of claim 1 or claim 2.

36. Process according to claim 35 wherein the hydrocarbon conversion process is cracking.

37. Process according to claim 35 wherein the hydrocarbon conversion process is hydrocracking.

38. Process according to claim 35 wherein the hydrocarbon conversion process is hydrogenation.

39. Process according to claim 35 wherein the hydrocarbon conversion process is polymerization.

40. Process according to claim 35 wherein the hydrocarbon conversion process is alkylation.

41. Process according to claim 35 wherein the hydrocarbon conversion process is reforming.

42. Process according to claim 35 wherein the hydrocarbon conversion process is hydrotreating.

43. Process according to claim 35 wherein the hydrocarbon conversion process is isomerization.

44. Process according to claim 43 wherein the isomerization conversion process is xylene isomerization.

45. Process according to claim 35 wherein the hydrocarbon conversion process is dehydrocyclization.

**Patentansprüche**

1. Kristalline Molekularsiebe mit dreidimensionalen mikroporösen Gerüststrukturen aus $MO_2$, $AlO_2^-$, $PO_2^+$ und $SiO_2$ tetraedrischen Oxideinheiten und mit einer empirischen chemischen Zusammensetzung auf wasserfreier Basis ausgedrückt durch die Formel:

$$mR: (M_wAl_xP_ySi_z)O_2$$

in der "R" wenigstens ein organisches Templat-Agens darstellt, das in dem intrakristallinen Porensystem anwesend ist; "m" die molare Menge von "R" darstellt, die pro Mol von $(M_wAl_xP_ySi_z)O_2$ anwesend ist und einen Wert von Null bis etwa 0,3 hat; "M" wenigstens zwei Elemente ausgewählt aus der Gruppe bestehend aus Arsen, Beryllium, Bor, Chrom, Kobalt, Gallium, Germanium, Eisen, Lithium, Magnesium, Mangan, Titan, Vanadium und Zink darstellt; "n" sein kann −3, −2, −1, 0 oder +1 abhängig von der Oxidationsstufe von "M" und "w", "x", "y" und "z" (worin "w" die Summe der einzelnen Molfraktionen "$W_1$", "$W_2$", "$W_3$" etc. ist und jedes Element eine Molfraktion von wenigstens 0,01 hat) jeweils die Molfraktionen der Elemente "M", Aluminium, Phosphor und Silicium darstellen, die als tetraedrische Oxide vorliegen, wobei diese einen solchen Wert haben, daß sie innerhalb des pentagonalen Zusammensetzungs-Gebiets liegen, das durch die Punkte A, B, C, D und E von Figur 1 definiert wird, wobei "w", "x", "y" und "z" die nachfolgenden Werte haben:

| | Mol-Fraktion | | |
|---|---|---|---|
| Punkt | x | y | (z+w) |
| A | 0,60 | 0-,37 | 0,03 |
| B | 0,37 | 0,60 | 0,03 |
| C | 0,01 | 0,60 | 0,39 |
| D | 0,01 | 0,01 | 0,98 |
| E | 0,60 | 0,01 | 0,39 |

2. Kristalline Molekularsiebe nach Ansprüche 1, bei denen "w", "x", "y" und "z" innerhalb des tetragonalen Zusammensetzungsgebiets liegen, das durch die Punkte a, b, c, d, e und f von Figur 2 definiert ist und wobei "w", "x", "y" und "z" die nachfolgenden Werte haben:

| | Mol-Fraktion | | |
|---|---|---|---|
| Punkt | x | y | (w+z) |
| a | 0,60 | 0,37 | 0,03 |
| b | 0,37 | 0,60 | 0,03 |
| c | 0,01 | 0,60 | 0,39 |
| d | 0,01 | 0,39 | 0,60 |
| e | 0,39 | 0,01 | 0,60 |
| f | 0,60 | 0,01 | 0,39 |

3. Kristalline Molekularsiebe nach Anspruch 1 oder 2, die ein charakteristisches Röntgenpulverbeugungsmuster haben, das wenigstens die in der nachfolgenden Tabelle aufgeführten d-Werte enthält:

| 2θ | d (Å) | relative Intensität |
|---|---|---|
| 7,2 - 7,7 | 12,28 - 11,48 | m-sst |
| 19,4 - 19,9 | 4,58 - 4,46 | schw-m |
| 20,85 - 21,3 | 4,26 - 4,17 | schw-sst |
| 22,1 - 22,6 | 4,02 - 3,93 | m-sst |
| 25,6 - 26,1 | 3,480 - 3,414 | sschw-m |

4. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intensität |
|---|---|---|
| 7,8 - 8,2 | 11,19 - 10,85 | m-st |
| 9,0 - 9,8 | 9,83 - 9,03 | sschw-sst |
| 12,8 - 13,6 | 6,92 - 6,51 | sschw-m |
| 19,9 - 20,5 | 4,46 - 4,33 | m-st |
| 20,8 - 21,8 | 4,27 - 4,08 | m-sst |
| 22,0 - 22,6 | 4,04 - 3,93 | m-sst |
| 22,6 - 23,1 | 3,93 - 3,85 | sschw-sst |
| 23,1 - 23,5 | 3,85 - 3,79 | schw-sst |

5. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intensität |
|---|---|---|
| 11,3 - 11,6 | 7,83 - 7,63 | schw-sst |
| 18,55 - 18,9 | 4,78 - 4,70 | sm-m |
| 21,85 - 22,2 | 4,07 - 4,00 | m-sst |
| 22,8 - 23,3 | 3,900 - 3,818 | schw-m |
| 26,4 - 27,3 | 3,370 - 3,267 | schw-m |
| 29,6 - 29,9 | 3,018 - 2,998 | schw-m |

6. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intensität |
|---|---|---|
| 13,8 - 14,2 | 6,42 - 6,23 | m-sst |
| 19,6 - 20,15 | 6,53 - 4,41 | m |
| 24,1 - 24,7 | 3,695 - 3,603 | m-sst |
| 27,9 - 28,6 | 3,198 - 3,121 | schw |
| 31,3 - 32,05 | 2,861 - 2,791 | schw |
| 34,35 - 35,0 | 2,610 - 2,601 | schw-m |

7. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intensität |
|---|---|---|
| 8,4 - 9,5 | 10,53 - 9,31 | schw-st |
| 20,2 - 20,4 | 4,40 - 4,35 | m |
| 22,0 - 22,1 | 4,040 - 4,022 | m |
| 22,5 - 22,7 | 3,952 - 3,92 | sst |
| 31,6 - 31,8 | 2,831 - 2,814 | schw-m |

8. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intensität |
|---|---|---|
| 9,3 - 9,8 | 9,51 - 9,03 | m-sst |
| 12,6 - 13,2 | 7,03 - 6,71 | schw-m |
| 15,8 - 16,3 | 5,61 - 5,44 | sschw-m |
| 20,25 - 21,2 | 4,39 - 4,19 | schw-sst |
| 24,8 - 25,4 | 3,59 - 3,507 | sschw-m |
| 30,0 - 30,9 | 2,979 - 2,894 | sschw-m |

9. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intentität |
|---|---|---|
| 10,6 - 11,1 | 8,35 - 7,97 | sschw-sst |
| 13,1 - 13,7 | 6,76 - 6,46 | sschw-sst |
| 17,0 - 17,6 | 5,22 - 5,04 | schw-st |
| 20,6 - 21,25 | 4,31 - 4,18 | sschw-m |
| 21,6 - 22,3 | 4,11 - 3,99 | m-sst |
| 28,1 - 28,8 | 3,175 - 3,100 | sst-m |

10. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intensität |
|---|---|---|
| 7,45 - 8,0 | 11,14 - 11,05 | sst |
| 8,1 - 8,3 | 10,91 - 10,65 | schw-m |
| 16,3 - 16,6 | 5,44 - 5,34 | schw-m |
| 18,9 - 19,4 | 4,70 - 4,57 | schw-m |
| 20,7 - 21,0 | 4,29 - 4,23 | schw-m |

11. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intensität |
|---|---|---|
| 9,2 - 9,6 | 9,61 - 9,21 | m |
| 13,1 - 13,5 | 6,76 - 6,56 | m |
| 17,8 - 18,4 | 4,98 - 4,82 | schw-m |
| 20,8 - 21,3 | 4,27 - 4,17 | m-sst |
| 22,2 - 22,85 | 4,00 - 3,892 | m-sst |
| 26,4 - 27,05 | 3,376 - 3,296 | schw-m |

12. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intensität |
|---|---|---|
| 12,3 - 12,95 | 7,20 - 6,83 | m-sst |
| 16,8 - 17,45 | 5,28 - 5,09 | sst-schw |
| 21,45 - 21,85 | 4,145 - 4,071 | m-sst |
| 27,1 - 27,85 | 3,291 - 3,232 | schw-sst |
| 32,4 - 33,2 | 2,763 - 2,699 | sschw-m |

13. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intensität |
|---|---|---|
| 9,2 - 9,6 | 9,61 - 9,21 | m-sst |
| 15,9 - 16,3 | 5,57 - 5,44 | sschw-m |
| 20,5 - 21,0 | 4,33 - 4,23 | m-sst |
| 24,3 - 25,1 | 3,66 - 3,548 | schw-m |
| 30,5 - 31,1 | 2,931 - 2,876 | sschw-m |

14. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intensität |
|---|---|---|
| 7,2 - 8,1 | 12,28 - 10,92 | sst |
| 12,9 - 13,6 | 6,86 - 6,51 | sschw |
| 21,2 - 22,2 | 4,19 - 4,501 | sschw-m |
| 22,5 - 23,45 | 3,95 - 3,793 | sschw-m |
| 26,6 - 27,9 | 3,351 - 3,198 | sschw-m |

15. Kristalline Molekularsiebe nach Anspruch 1 oder 2, die ein charakteristisches Röntgenpulverbeugungsmuster haben, das wenigstens die nachfolgenden d-Werte enthält:

| 2θ | d (Å) | relative Intensität |
|---|---|---|
| 9,4 - 9,6 | 9,41 - 9,21 | sst |
| 12,8 - 13,1 | 6,92 - 6,76 | sschw-m |
| 16,0 - 16,3 | 5,54 - 5,44 | sschw-m |
| 20,5 - 21,0 | 4,31 - 4,23 | m-sst |
| 24,6 - 25,3 | 3,613 - 3,526 | sm-m |
| 30,6 - 31,1 | 2,921 - 2,876 | sschw-m |

16. Kristalline Molekularsiebe nach Anspruch 1 oder 2, bei denen "M" ausgewählt wird aus der Gruppe bestehend aus Magnesium, Mangan, Titan, Vanadium, Zink, Kobalt, Eisen und Beryllium.

17. Kristalline Molekularsiebe nach Anspruch 1 oder 2, bei denen zwei Elemente "M" anwesend sind.

18. Kristalline Molekularsiebe nach Anspruch 17, bei denen "M" Kobalt und Mangan ist.

19. Kristalline Molekularsiebe nach Anspruch 1 oder 2, bei denen drei Elemente "M" anwesend sind.

20. Kristalline Molekularsiebe nach Anspruch 19, bei denen "M" Kobalt, Mangan und Magnesium ist.

21. Verfahren zur Herstellung der kristallinen Molekularsiebe nach Anspruch 1, die dreidimensionale Gerüststrukturen haben, wobei dieses Verfahren umfaßt das Erhitzen auf eine effektive Temperatur zwischen 50°C und 250°C einer Reaktionsmischung mit einer Zusammensetzung zahlenmäßig ausgedrückt durch die nachfolgenden molaren Verhältnisse der Oxide:

$$aR : (M_wAl_xP_ySi_z)O_2 : bH_2O$$

wobei "R" ein organisches Templat-Agens ist; "a" die Menge an "R" ist und Null oder eine effektive Menge größer als 0 bis etwa 6 sein kann; "b" einen Wert von Null bis etwa 500 hat; "M" wenigstens zwei Elemente ausgewählt aus der Gruppe bestehend aus Arsen, Beryllium, Bor, Chrom, Kobalt, Gallium, Germanium, Eisen, Lithium, Magnesium, Mangan, Titan, Vanadium und Zink darstellt und "w", "x", "y" und "z" jeweils die Molfraktionen der Elemente "M", Aluminium und Phosphor und Silicium, in dem $(M_wAl_xP_ySi_z)O_2$-Bestandteil darstellen, und wobei die Molfraktionen jedes Elements Aluminium, Phosphor und Silicium jeweils einen Wert von wenigstens 0,01 haben, für eine Zeitdauer, die ausreicht, um Kristalle der Molekularsiebe gemäß Anspruch 1 herzustellen.

22. Verfahren nach Anspruch 21, bei dem "w", "x", "y" und "z" innerbalb des durch die Punkte F, G, H, I und J von Figur 3 definierten Bereichs liegen und "w", "x", "y" und "z" die nachfolgenden Werte haben:

| | Mol-Fraktion | | |
|---|---|---|---|
| Punkt | x | y | (z+w) |
| F | 0,60 | 0,37 | 0,03 |
| G | 0,37 | 0,60 | 0,03 |
| H | 0,01 | 0,60 | 0,39 |
| I | 0,01 | 0,01 | 0,98 |
| J | 0,60 | 0,01 | 0,39 |

23. Verfahren nach Anspruch 21, bei dem die Quelle für Phosphor in der Reaktionsmischung Orthophosphorsäure ist.

24. Verfahren nach Anspruch 21, bei dem die Quelle für Phosphor in der Reaktionsmischung Orthophosphorsäure ist und die Quelle für Aluminium wenigstens eine Verbindung ausgewählt aus der Gruppe bestehend aus Pseudo-Boehmit und Aluminiumalkoxid ist.

25. Verfahren nach Anspruch 24, bei dem das Aluminiumalkoxid Aluminiumisopropyloxid ist.

26. Verfahren nach Anspruch 21, bei dem die Quelle für Silicium Siliciumdioxid ist.

27. Verfahren nach Anspruch 21, bei dem die reaktiven Quellen für die Elemente "M" ausgewählt werden aus der Gruppe bestehend aus den Oxiden, Hydroxiden, Alkoxiden, Nitraten, Sulfaten, Boraten, Germanaten, Vanadaten, Halogeniden, Carboxylaten sowie deren Mischungen.

28. Verfahren nach Anspruch 21 oder Anspruch 22, bei dem das organische Templat-Agens eine quaternäre Ammonium- oder quaternäre Phosphonium-Verbindung ist mit der Formel:

$$R_4X^+$$

in der X Stickstoff oder Phosphor ist und R jeweils eine Alkyl- oder Arylgruppe mit 1 bis 8 Kohlenstoffatomen ist.

29. Verfahren nach Anspruch 21, bei dem das organische Templat-Agens ein Amin ist.

30. Verfahren nach Anspruch 21 oder Anspruch 22, bei dem das Templat-Agens ausgewählt wird aus der Gruppe bestehend aus dem Tetrapropylammoniumion; Tetraethylammoniumion; Tripropylamin; Triethylamin; Triethanolamin; Piperidin; Cyclohexylamin; 2-Methylpyridin; N,N-Dimethylbenzylamin; N,N-Dimethylethanolamin; Chlor; N,N-Dimethylpiperazin; 1,4-Diazabicyclo-(2,2,2)-octan; N-Methyldiethanolamin; N-Methylethanolamin; N-Methylpiperidin; 3-Methylpiperidin; N-Methylcyclohexylamin; 3-Methylpyridin; 4-Methylpyridin; Chinuclidin; N,N'-Dimethyl-1,4-Diazabicyclo(2,2,2)octanion; Tetramethylammoniumion; Tetrabutylammoniumion; Tetrapentylammoniumion; Di-n-Butylamin; Neopentylamin; Di-n-pentylamin; Isopropylamin; tertiär-Butylamin; Ethylendiamin; Pyrrolidin; 2-Imidazolidon; Di-n-propylamin und einem polymeren quaternären Ammoniumsalz $[(C_{14}H_{32}N_2)(OH)_2]_x$, worin x einen Wert von wenigstens 2 hat.

31. Molekularsiebe hergestellt durch Calcinieren der Zusammensetzungen gemäß Anspruch 1 oder Anspruch 2, bei einer Temperatur, die ausreichend hoch ist, um wenigstens etwas von dem organischen Templat-Agens, das in dem intrakristallinen Porensystem anwesend ist, zu entfernen.

32. Verfahren zur Trennung molekularer Spezies aus einem Gemisch mit molekularen Spezies, die einen geringeren Polaritätsgrad aufweisen, umfassend das in Kontakt bringen dieses Gemischs molekularer Spezies mit einem Molekularsieb gemäß Anspruch 1 oder Anspruch 2, das Porendurchmesser aufweist, die weit genug sind, um wenigstens eine der polareren molekularen Spezies zu adsorbieren, wobei dieses Molekularsieb wenigstens teilweise aktiviert wird, wobei Moleküle der polareren molekularen Spezies selektiv in das intrakristalline Porensystem des Molekularsiebs adsorbiert werden.

33. Verfahren zur Trennung eines Gemischs molekularer Spezies mit unterschiedlichen kinetischen Durchmessern, umfassend das in Kontakt bringen dieses Gemischs mit einem Molekularsieb gemäß Anspruch 1 oder Anspruch 2, das Porendurchmesser aufweist, die weit genug sind, um wenigstens eine jedoch nicht alle dieser molekularen Spezies dieser Mischung zu adsorbieren, wobei das Molekularsieb wenigstens teilweise aktiviert wird, wobei wenigstens einige Moleküle, deren kinetische Durchmesser ausreichend kleine sind, in das intrakristalline Porensystem des Molekularsiebs eintreten können.

34. Verfahren nach Anspruch 32, bei dem die polarere molekulare Spezies Wasser ist.

35. Verfahren zur Umwandlung eines Kohlenwasserstoffs, umfassend das in Kontakt bringen dieses Kohlenwasserstoffs unter Kohlenwasserstoff-Umwandlungs-Bedingungen mit einem Molekularsieb gemäß Anspruch 1 oder Anspruch 2.

36. Verfahren nach Anspruch 35, bei dem der Kohlenwasserstoff-Umwandlungsprozeß "Cracking" ist.

37. Verfahren nach Anspruch 35, bei dem der Kohlenwasserstoff-Umwandlungsprozeß "Hydrocracking" ist.

38. Verfahren nach Anspruch 35, bei dem der Kohlenwasserstoff-Umwandlungsprozeß eine Hydrogenierung ist.

39. Verfahren nach Anspruch 35, bei dem der Kohlenwasserstoff-Umwandlungsprozeß eine Polymerisierung ist.

40. Verfahren nach Anspruch 35, bei dem der Kohlenwasserstoff-Umwandlungsprozeß eine Alkylierung ist.

41. Verfahren nach Anspruch 35, bei dem der Kohlenwasserstoff-Umwandlungsprozeß Reformieren ist.

42. Verfahren nach Anspruch 35, bei dem der Kohlenwasserstoff-Umwandlungsprozeß eine Hydrobehandlung ist.

43. Verfahren nach Anspruch 35, bei dem der Kohlenwasserstoff-Umwandlungsprozeß eine Isomerisierung ist.

44. Verfahren nach Anspruch 43, bei dem der Isomerisierungs-Umwandlungsprozeß eine Xylenisomerisierung ist.

45. Verfahren nach Anspruch 35, bei dem der Kohlenwasserstoff-Umwandlungsprozeß eine Dehydrocyclisierung ist.

**Revendications**

1. Tamis moléculaires cristallins ayant des ossatures structurales microporeuses tridimensionnelles formées de motifs d'oxydes tétraédriques $MO_2$, $AlO_2^-$ et $PO_2^-$ et $SiO_2$ et dont la composition chimique sur

base anhydre s'exprime par la formule empirique:

$$mR : (M_wAl_xP_ySi_z)O_2$$

dans laquelle "R" représente au moins un agent organique d'orientation structurale présent dans le système de pores intracristallins; "m" représente la quantité molaire de "R" par mole de $(M_wAl_xP_ySi_z)O_2$ et a une valeur de zéro (0) à environ 0,3; "M" représente au moins deux éléments choisis dans le groupe comprenant l'arsenic, le béryllium, le bore, le chrome, le cobalt, le gallium, le germanium, le fer, le lithium, le magnésium, le manganèse, le titane, le vanadium et le zinc; "n" peut avoir la valeur −3, −2, −1, 0 ou +1 selon le degré d'oxydation de "M" et "w", "x", "y" et "z" ("w" représentant la somme des fractions molaires individuelles de "$W_1$", "$W_2$" et "$W_3$", etc., et chaque élément ayant une fraction molaire d'au moins 0,01) représentent respectivement les fractions molaires des éléments "M", aluminium, phosphore et silicium, présents à l'état d'oxydes tétraèdriques, ces paramètres étant choisis de manière qu'ils rentrent dans l'aire pentagonale de composition délimitée par les points A, B, C, D et E de la Figure 1, "w", "x", "y" et "z" ayant les valeurs suivantes:

| | Fraction molaire | | |
|---|---|---|---|
| Point | x | y | (z + w) |
| A | 0,60 | 0,37 | 0,03 |
| B | 0,37 | 0,60 | 0,03 |
| C | 0,01 | 0,60 | 0,39 |
| D | 0,01 | 0,01 | 0,98 |
| E | 0,60 | 0,01 | 0,39 |

2. Tamis moléculaires cristallins suivant la revendication 1, dans lesquels "w", "x", "y' et "z" rentrent dans l'aire tétragonale de composition délimitée par les points a, b, c, d, e et f de la Figure 2 et "w", "x", "y" et "z" ont les valeurs suivantes:

| | Fraction molaire | | |
|---|---|---|---|
| Point | x | y | (w + z) |
| a | 0,60 | 0,37 | 0,03 |
| b | 0,37 | 0,60 | 0,03 |
| c | 0,01 | 0,60 | 0,39 |
| d | 0,01 | 0,39 | 0,60 |
| e | 0,39 | 0,01 | 0,60 |
| f | 0,60 | 0,01 | 0,39 |

3. Tamis moléculaires cristallins suivant la revendication 1 ou 2, ayant un diagramme caractéristique de diffraction des rayons X sur poudre qui contient au moins les distances d indiquées sur le tableau suivant:

| 2θ | d (Å) | Intensité relative |
|---|---|---|
| 7,2 - 7,7 | 12,28 - 11,48 | m-vs |
| 19,4 - 19,9 | 4,58 - 4,46 | w-m |
| 20,85 - 21,3 | 4,26 - 4,17 | w-vs |
| 22,1 - 22,6 | 4,02 - 3,93 | m-vs |
| 25,6 - 26,1 | 3,480 - 3,414 | vw-m |

4. Tamis moléculaires suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 7,8 - 8,2 | 11,19 - 10,85 | m-s |
| 9,0 - 9,8 | 9,83 - 9,03 | vw-vs |
| 12,8 - 13,6 | 6,92 - 6,51 | vw-m |
| 19,9 - 20,5 | 4,46 - 4,33 | m-s |
| 20,8 - 21,8 | 4,27 - 4,08 | m-vs |
| 22,0 - 22,6 | 4,04 - 3,93 | m-vs |
| 22,6 - 23,1 | 3,93 - 3,85 | vw-vs |
| 23,1 - 23,5 | 3,85 - 3,79 | w-vs |

5. Tamis moléculaires cristallins suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 11,3 - 11,6 | 7,83 - 7,63 | w-vs |
| 18,55 - 18,9 | 4,78 - 4,70 | vm-m |
| 21,85 - 22,2 | 4,07 - 4,00 | m-vs |
| 22,8 - 23,3 | 3,900 - 3,818 | w-m |
| 26,4 - 27,3 | 3,370 - 3,267 | w-m |
| 29,6 - 29,9 | 3,018 - 2,988 | w-m |

6. Tamis moléculaires cristallins suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 13,8 - 14,2 | 6,42 - 6,23 | m-vs |
| 19,6 - 20,15 | 6,53 - 4,41 | m |
| 24,1 - 24,7 | 3,695 - 3,603 | m-vs |
| 27,9 - 28,6 | 3,198 - 3,121 | w |
| 31,3 - 32,05 | 2,861 - 2,791 | w |
| 34,35 - 35,0 | 2,610 - 2,601 | w-m |

7. Tamis moléculaires cristallins suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 8,4 - 9,5 | 10,53 - 9,31 | w-s |
| 20,2 - 20,4 | 4,40 - 4,35 | m |
| 22,0 - 22,1 | 4,040 - 4,022 | m |
| 22,5 - 22,7 | 3,952 - 3,92 | vs |
| 31,6 - 31,8 | 2,831 - 2,814 | w-m |

8. Tamis moléculaires cristallins suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 9,3 - 9,8 | 9,51 - 9,03 | m-vs |
| 12,6 - 13,2 | 7,03 - 6,71 | w-m |
| 15,8 - 16,3 | 5,61 - 5,44 | vw-m |
| 20,25 - 21,2 | 4,39 - 4,19 | w-vs |
| 24,8 - 25,4 | 3,59 - 3,507 | vw-m |
| 30,0 - 30,9 | 2,979 - 2,894 | vw-m |

9. Tamis moléculaires cristallins suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 10,6 - 11,1 | 8,35 - 7,97 | vw-vs |
| 13,1 - 13,7 | 6,76 - 6,46 | vw-vs |
| 17,0 - 17,6 | 5,22 - 5,04 | w-s |
| 20,6 - 21,25 | 4,31 - 4,18 | vw-m |
| 21,6 - 22,3 | 4,11 - 3,99 | m-vs |
| 28,1 - 28,8 | 3,175 - 3,100 | vw-m |

10. Tamis moléculaires cristallins suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 7,45 - 8,0 | 11,14 - 11,05 | vs |
| 8,1 - 8,3 | 10,91 - 10,65 | w-m |
| 16,3 - 16,6 | 5,44 - 5,34 | w-m |
| 18,9 - 19,4 | 4,70 - 4,57 | w-m |
| 20,7 - 21,0 | 4,29 - 4,23 | w-m |

11. Tamis moléculaires cristallins suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 9,2 - 9,6 | 9,61 - 9,21 | m |
| 13,1 - 13,5 | 6,76 - 6,56 | m |
| 17,8 - 18,4 | 4,98 - 4,82 | w-m |
| 20,8 - 21,3 | 4,27 - 4,17 | m-vs |
| 22,2 - 22,85 | 4,00 - 3,892 | m-vs |
| 26,4 - 27,05 | 3,376 - 3,296 | w-m |

12. Tamis moléculaires cristallins suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 12,3 - 12,95 | 7,20 - 6,83 | m-vs |
| 16,8 - 17,45 | 5,28 - 5,09 | vw-w |
| 21,45 - 21,85 | 4,145 - 4,071 | m-vs |
| 27,1 - 27,85 | 3,291 - 3,232 | w-vs |
| 32,4 - 33,2 | 2,763 - 2,699 | vw-m |

13. Tamis moléculaires cristallins suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 9,2 - 9,6 | 9,61 - 9,21 | m-vs |
| 15,9 - 16,3 | 5,57 - 5,44 | vw-m |
| 20,5 - 21,0 | 4,33 - 4,23 | m-vs |
| 24,3 - 25,1 | 3,66 - 3,548 | w-m |
| 30,5 - 31,1 | 2,931 - 2,876 | vw-m |

14. Tamis moléculaires cristallins suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 7,2 - 8,1 | 12,28 - 10,92 | vs |
| 12,9 - 13,6 | 6,86 - 6,51 | vw |
| 21,2 - 22,2 | 4,19 - 4,501 | vw-m |
| 22,5 - 23,45 | 3,95 - 3,793 | vw-m |
| 26,6 - 27,9 | 3,351 - 3,198 | vw-m |

15. Tamis moléculaires cristallins suivant la revendication 1 ou 2, ayant un diagramme caractéristique de diffraction des rayons X sur poudre qui contient au moins les distances d suivantes:

| 2θ | d (Å) | Intensité relative |
|---|---|---|
| 9,4 - 9,6 | 9,41 - 9,21 | vs |
| 12,8 - 13,1 | 6,92 - 6,76 | vw-m |
| 16,0 - 16,3 | 5,54 - 5,44 | vw-m |
| 20,5 - 21,0 | 4,31 - 4,23 | m-vs |
| 24,6 - 25,3 | 3,613 - 3,526 | vm-m |
| 30,6 - 31,1 | 2,921 - 2,876 | vw-m |

16. Tamis moléculaires cristallins suivant la revendication 1 ou 2, dans lesquels "M" est choisi dans le groupe comprenant le magnésium, le manganèse, le titane, le vanadium, le zinc, le cobalt, le fer et le béryllium.

17. Tamis moléculaires cristallins suivant la revendication 1 ou 2, dans lesquels deux éléments "M" sont présents.

18. Tamis moléculaires cristallins suivant la revendication 17, dans lesquels "M" est le cobalt et le manganèse.

19. Tamis moléculaires cristallins suivant la revendication 1 ou 2, dans lesquels trois éléments "M" sont présents.

20. Tamis moléculaires cristallins suivant la revendication 19, dans lesquels "M" est le cobalt, le manganèse et le magnésium.

21. Procédé de production des tamis moléculaires cristallins suivant la revendication 1 ayant des ossatures structurales tridimensionnelles, procédé qui consiste à chauffer à une température efficace entre 50°C et 250°C, un mélange réactionnel dont la composition, exprimée par les rapports molaires des oxydes, répond à la formule suivante:

$$aR : (M_wAl_xP_ySi_z)O_2 : bH_2O$$

dans laquelle "R" est un agent organique d'orientation structurale; "a" est la quantité de "R" et peut être égal à zéro ou à une quantité effective allant de plus de zéro à environ 6; "b" a une valeur de zéro à environ 500; "M" représente au moins deux éléments choisis dans le groupe comprenant l'arsenic, le béryllium, le bore, le chrome, le cobalt, le gallium, le germanium, le fer, le lithium, le magnésium, le manganèse, le titane, le vanadium et le zinc; et "w", "x", "y" et "z" représentent les fractions molaires respectives d'éléments "M", d'aluminium et de phosphore et de silicium dans le constituant $(M_wAl_xP_ySi_z)O_2$ et la fraction molaire de chaque élément, d'aluminium, de phosphore et de solicium a une valeur d'au moins 0,01, pendant une période efficace pour produire des cristaux des tamis moléculaires suivant la revendication 1.

22. Procédé suivant la revendication 21, dans lequel "w", "x", "y" et "z" rentrent dans l'aire délimitée par les points F, G, H, I, et J de la Figure 3 et "w", "x", "y" et "z" ont les valeurs suivantes:

| | Fraction molaire | | |
|---|---|---|---|
| Point | x | y | (z+w) |
| F | 0,60 | 0,37 | 0,03 |
| G | 0,37 | 0,60 | 0,03 |
| H | 0,01 | 0,60 | 0,39 |
| I | 0,01 | 0,01 | 0,98 |
| J | 0,60 | 0,01 | 0,39 |

23. Procédé suivant la revendication 21, dans lequel la source de phosphore dans le mélange réactionnel est l'acide orthophosphorique.

24. Procédé suivant la revendication 21, dans lequel la source de phosphore dans le mélange réactionnel est l'acide orthophosphorique et la source d'aluminium est au moins un composé choisi dans le groupe de la pseudoboehmite et d'un alcoolate d'aluminium.

25. Procédé suivant la revendication 24, dans lequel l'alcoolate d'aluminium est l'isopropylate d'aluminium.

26. Procédé suivant la revendication 21, dans lequel la source de silicium est la silice.

27. Procédé suivant la revendication 21, dans lequel les sources réactives d'éléments "M" sont choisies dans le groupe comprenant des oxydes, des hydroxydes, des alcoolates, des nitrates, des sulfates, des borates, des germanates, des vanadates, des halogénures, des carboxylates et leurs mélanges.

28. Procédé suivant la revendication 21 ou la revendication 22, dans lequel l'agent organique d'orientation structurale est un composé d'ammonium quaternaire ou de phosphonium quaternaire répondant à la formule:

$$R_4X^+$$

dans laquelle X est l'azote ou le phosphore et chaque R est un groupe alkyle ou aryle contenant 1 à 8 atomes de carbone.

29. Procédé suivant la revendication 21, dans lequel l'agent organique d'orientation structurale est une amine.

30. Procédé suivant la revendication 21 ou la revendication 22, dans lequel l'agent d'orientation structurale est choisi dans le groupe comprenant l'ion tétrapropylammonium; l'ion tétraéthylammonium; la tripropylamine; le triéthylamine; la triéthanolamine; la pipéridine; la cyclohexylamine; la 2-méthylpyridine; la N,N-diméthylbenzylamine; la N,N-diméthyléthanolamine; le chlore; la N,N-diméthylpipérazine; le 1,4-diazabicyclo-(2,2,2)octane; la N-méthyldiéthanolamine; la N-méthyléthanolamine; la N-méthylpipéridine; la 3-méthylpipéridine; la N-méthylcyclohexylamine; la 3-méthylpyridine; la 4-méthylpyridine; la quinuclidine; l'ion N,N-diméthyl-1,4-diazabicyclo-(2,2,2)-octane; l'ion tétraméthylammonium; l'ion tétrabutylammonium; l'ion tétrapentylammonium; la di-n-butylamine; la néopentylamine; la di-n-pentylamine; l'isopropylamine; la tertio-butylamine; l'éthylènediamine; la pyrrolidine; la 2-imidazolidone; la di-n-propylamine; et un sel d'ammonium quaternaire polymérique de formule $[(C_{14}H_{32}N_2)(OH)_2]x$ dans laquelle x a une valeur d'au moins 2.

31. Tamis moléculaires préparés par calcination des compositions de la revendication 1 ou de la revendication 2 à une température suffisamment haute pour éliminer au moins une partie de tout agent organique d'orientation structurale présent dans le système de pores intracristallins.

32. Procédé de séparation d'espèces moléculaires d'un mélange avec des espèces moléculaires ayant un plus faible degré de polarité, qui consiste à faire entrer ledit mélange d'espèces moléculaires en contact avec un tamis moléculaire suivant la revendication 1 ou la revendication 2 ayant des diamètres de pores suffisamment grands pour adsorber au moins l'une des espèces moléculaires plus polaires, ledit tamis moléculaire étant au moins partiellement activé de manière que les molécules des espèces moléculaires plus polaires soient sélectivement adsorbées dans son système de pores intracristallins.

33. Procédé pour séparer un mélange d'espèces moléculaires ayant des diamètres cinétiques différents, qui consiste à faire entrer ledit mélange en contact avec un tamis moléculaire suivant la revendication 1 ou la revendication 2 ayant des diamètres de pores assez grands pour adsorber au moins l'une mais non la totalité des espèces moléculaires dudit mélange, le tamis moléculaire étant au moins partiellement activé de manière qu'au moins certaines molécules, dont les diamètres cinétiques sont suffisamment petits, puissent pénétrer dans son système de pores intracristallins.

34. Procédé suivant la revendication 32, dans lequel l'espèce moléculaire plus polaire est l'eau.

35. Procédé de transformation d'un hydrocarbure, qui consiste à faire entrer ledit hydrocarbure, dans des conditions propices à sa transformation, en contact avec un tamis moléculaire suivant la revendication 1 ou la revendication 2.

36. Procédé suivant la revendication 35, dans lequel la transformation d'hydrocarbures est un craquage.

37. Procédé suivant la revendication 35, dans lequel la transformation d'hydrocarbures est un hydrocraquage.

38. Procédé suivant la revendication 35, dans lequel la transformation d'hydrocarbures est une hydrogénation.

39. Procédé suivant la revendication 35, dans lequel la transformation d'hydrocarbures est une polymérisation.

40. Procédé suivant la revendication 35, dans lequel la transformation d'hydrocarbures est une alkylation.

41. Procédé suivant la revendication 35, dans lequel la transformation d'hydrocarbures est un reformage.

42. Procédé suivant la revendication 35, dans lequel la transformation d'hydrocarbures est un hydrotraitement.

43. Procédé suivant la revendication 35, dans lequel la transformation d'hydrocarbures est une isomérisation.

44. Procédé suivant la revendication 43, dans lequel la transformation par isomérisation est l'isomérisation de xylène.

45. Procédé suivant la revendication 35, dans lequel la transformation d'hydrocarbures est une déshydrocyclisation.

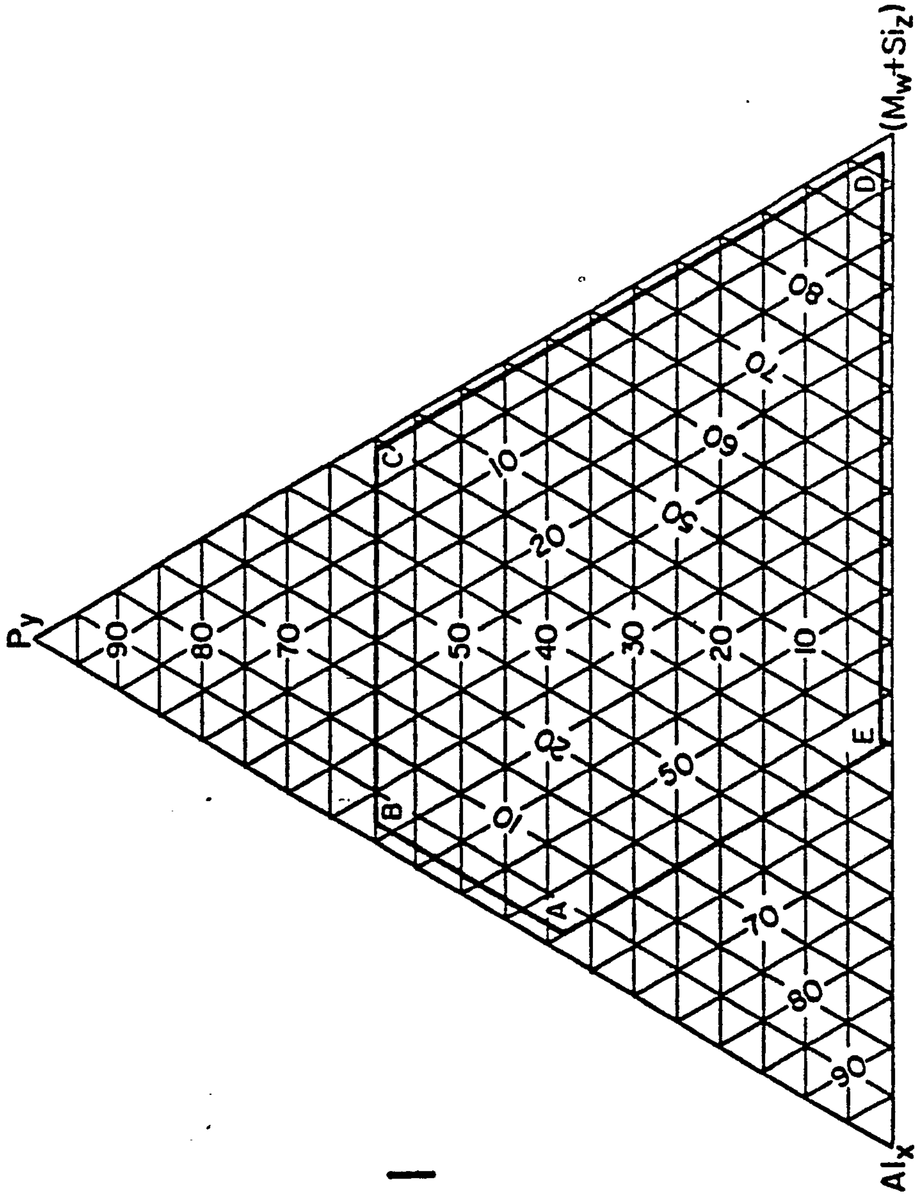


FIG. 1

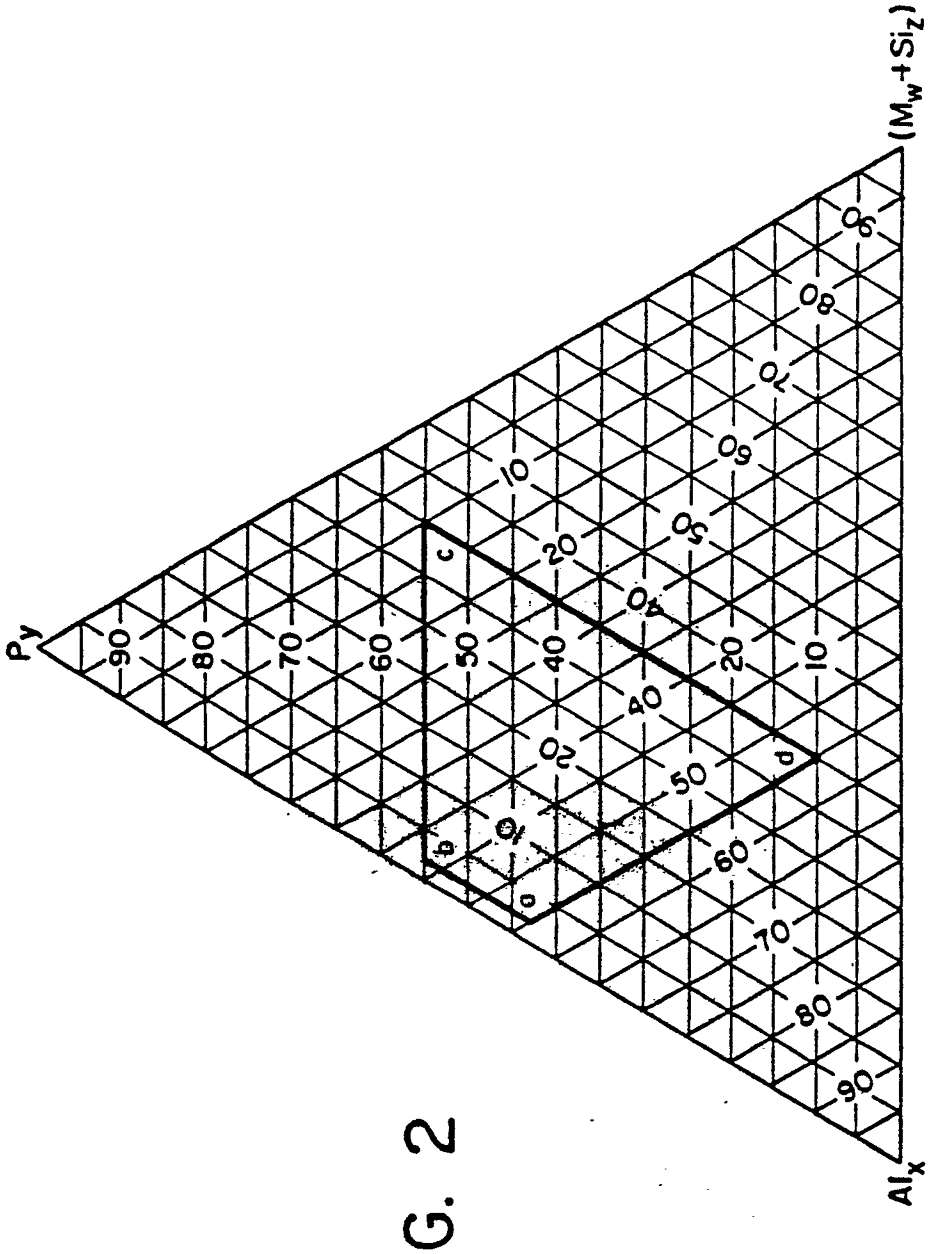


FIG. 2

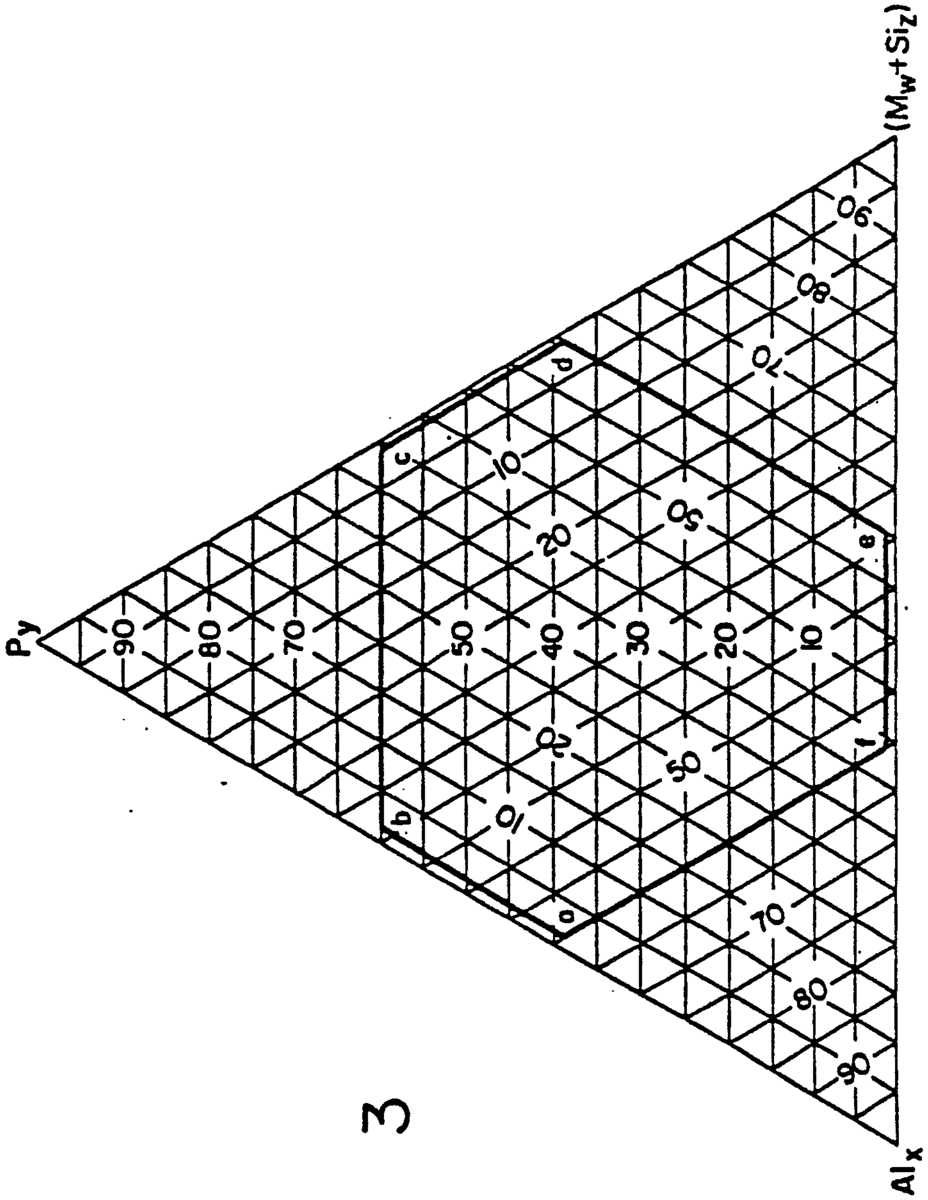


FIG. 3

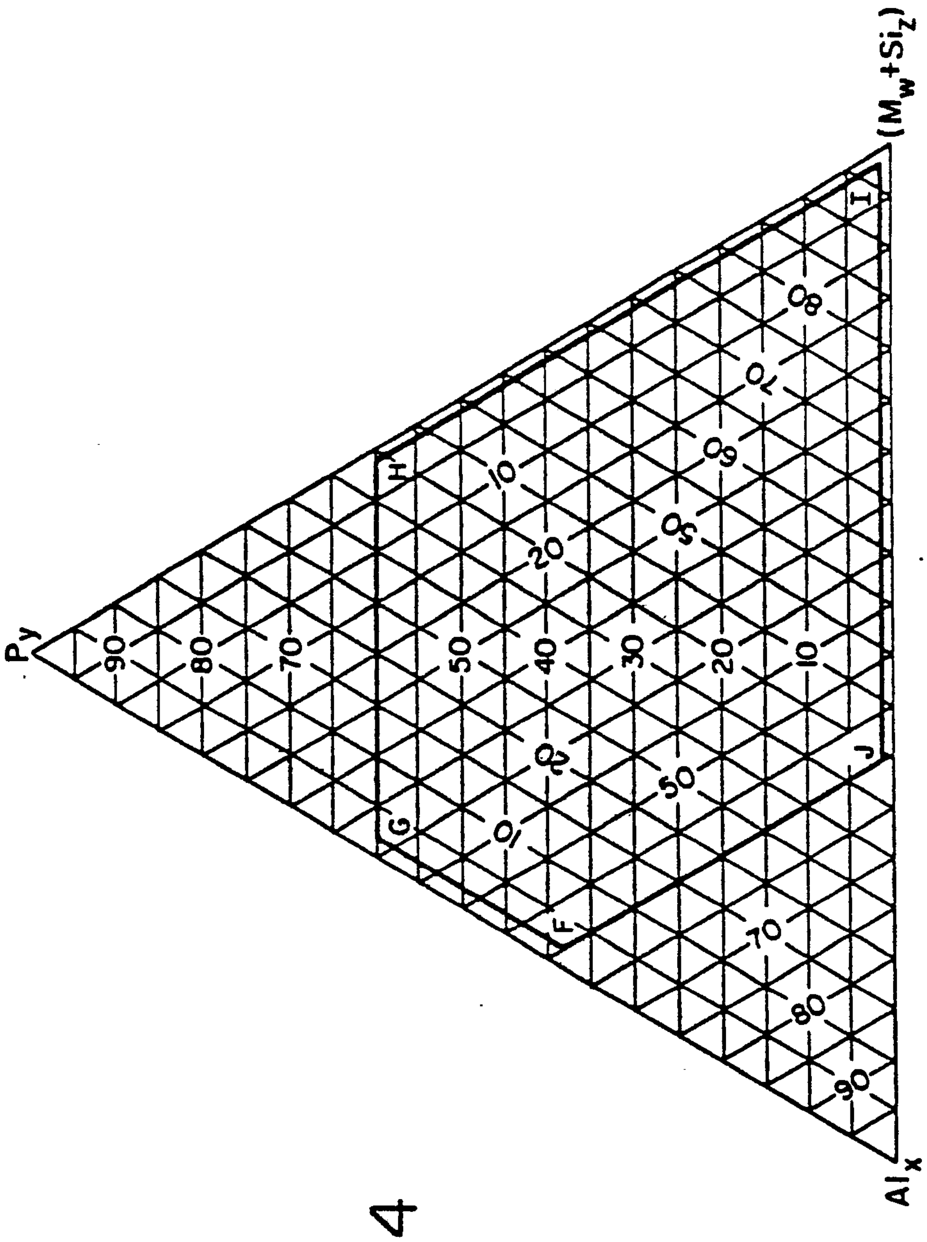


FIG. 4